# EUROPEAN PATENT SPECIFICATION

(11) **EP 1 809 303 B1**
(45) Date of publication and mention of the grant of the patent: **06.03.2019**
(21) Application number: 05788866.1
(22) Date of filing: 23.09.2005
(51) Int. Cl.: A61K 31/737, A61K 36/03, A61P 17/02, A61P 29/00, A61P 43/00, A61P 41/00, A61K 9/70, A61K 47/36, A61L 31/04, C08L 5/00

(54) **PHARMACEUTICAL COMPOSITIONS AND METHODS RELATING TO INHIBITING FIBROUS ADHESIONS OR INFLAMMATORY DISEASE USING LOW SULPHATE FUCANS**
PHARMAZEUTISCHE ZUSAMMENSETZUNGEN UND VERFAHREN ZUR HEMMUNG VON FIBRÖSER ADHÄSION ODER ENTZÜNDUNGSERKRANKUNG MIT NIEDERMOLEKULAREN SULFAT-FUCANEN
COMPOSITIONS PHARMACEUTIQUES ET METHODES RELATIVES A L'INHIBITION D'ADHERENCES FIBREUSES OU DE MALADIES INFLAMMATOIRES A L'AIDE DE FUCANES A BASSE TENEUR EN SULFATE

(30) Priority: 23.09.2004 US 612665 P; 23.09.2004 US 612676 P
(43) Date of publication of application: 25.07.2007
(73) Proprietor: ARC Medical Devices, Inc., Vancouver BC V6T 1Z3 (CA)
(72) Inventor: CASHMAN, Johanne, Vancouver, British Columbia V6N 1R1 (CA); SPRINGATE, Christopher, Vancouver, British Columbia V5T 2H5 (CA); WINTERNITZ, Charles, Colorado Springs CO 80918-4757 (CA)
(74) Representative: Stolmár & Partner Patentanwälte PartG mbB
(86) International application number: PCT/CA2005/001446
(87) International publication number: WO 2006/032143

(56) References cited:
- WO-A-2004/105737
- WO-A-2006/020994
- WO-A1-00/56283
- WO-A1-97/24459
- WO-A1-2004/020473
- CA-A1- 2 223 573
- CA-A1- 2 367 107
- CA-A1- 2 458 853
- CA-C- 1 297 791
- CA-C- 1 330 298
- US-A- 5 534 261
- GUVENAL T ET AL: 'Prevention of postoperative adhesion formation in rat uterine horn model by nimesulide: a selective COX-2 inhibitor.' HUMAN REPRODUCTION. vol. 16, no. 8, 2001, pages 1732 - 1735, XP002485910
- RISBERG B.: 'Adhesion: Preventive Strategies.' EUR J OF SURGERY. vol. 577, 1997, pages 32 - 39, XP008117467
- STEINLEITNER A; LAMBERT H; HENDERSON S.: 'New Modalities Under Development for Adhesion Prevention: Immunomodulatory Agents and Poloxamer Barrier Materials.' GYNECOLOGIC SURGERY AND ADHESION PREVENTION. 1993, pages 235 - 251
- MOLLOY RG ET AL: 'Reduction of the Local Toxicity of Intraperitoneal Chemotherapy; an Experimental Model.' INTRAPARITONEAL CHEMOTHERAPY. vol. 159, no. 6, 1990, pages 175 - 177
- STANGEL J J; NISBET J D; SETTLES H.: 'Formation and Prevention of Postoperative Abdominal Adhesions.' J REPROD MED. vol. 29, no. 3, 1984, pages 143 - 156, XP009074232
- HOLTZ G, BAKER E, TSAI C.: 'Effect of Thirty-two Per Cent Dextran 70 on Peritoneal Adhesion Formation and Re-Formation After Lysis.' FERTILITY AND STERILITY. vol. 33, no. 6, 1980, pages 660 - 662, XP008117468

## Description

### CROSS-REFERENCE TO OTHER APPLICATIONS

The present application claims priority from United States provisional patent application No. 60/612,676, filed September 23, 2004; and, United States provisional patent application No. 60/612,665.

### TABLE OF CONTENTS

The following is a Table of Contents to assist review of the present application:
CROSS-REFERENCE TO OTHER APPLICATIONS
TABLE OF CONTENTS
BACKGROUND
SUMMARY
BRIEF DESCRIPTION OF THE FIGURES
DETAILED DESCRIPTION
   General Discussion Of Exemplary Anti-Fibrous Adhesion Agents
   Fucans
   Films
   Gels
   Instillates
   Anti-SDF-1 Agents
   Discussion Of Quantitative Effectiveness Of Anti-Fibrous Adhesion
   Agents
EXAMPLES
SEQUENCE LISTING
CLAIMS
ABSTRACT

### BACKGROUND

A fibrous adhesion is a type of scar that forms between two parts of the body, usually after surgery (surgical adhesion). Fibrous adhesions can cause severe problems. For example, fibrous adhesions involving the female reproductive organs (ovaries, Fallopian tubes) can cause infertility, dyspareunia and severe pelvic pain. Fibrous adhesions that occur in the bowel can cause bowel obstruction or blockage, and fibrous adhesions can also form in other places such as around the heart, spine and in the hand. In addition to surgery, fibrous adhesions can be caused for example by endometriosis, infection, chemotherapy, radiation, trauma and cancer.

A variety of fibrous adhesions are discussed in this document. Terms such as surgical adhesions, post-surgical adhesions, postoperative adhesions, adhesions due to pelvic inflammatory disease, adhesions due to mechanical injury, adhesions due to radiation, adhesions due to radiation treatment, adhesions due to trauma, and adhesions due to presence of foreign material all refer to adherence of tissues to each other due to a similar mechanism and are all included in the term fibrous adhesions.

Fibrous adhesion formation is a complex process in which tissues that are normally separated in the body grow into each other. Surgical adhesions (also known as post-surgical adhesions) develop from the otherwise normal wound healing response of the tissues to trauma and have been reported to occur in over two-thirds of all abdominal surgical patients (Ellis, H., Surg. Gynecol. Obstet. 133: 497 (1971)). The consequences of these fibrous adhesions are varied and depend upon the surgical site or other site, such as a disease site, involved. Problems may include chronic pain, obstruction of the intestines and even an increased risk of death after cardiac surgery (diZerega, G. S., Prog. Clin. Biol. Res. 381: 1-18 (1993); diZerega, G. S., Fertil. Steril. 61:219-235 (1994); Dobell, A. R., Jain, A. K., Ann. Thorac. Surg. 37: 273-278 (1984)). In women of reproductive age, fibrous adhesions involving the uterus, fallopian tubes or ovaries are estimated to account for approximately 20% of all infertility cases (Holtz, G., Fertil. Steril. 41: 497-507 (1984); Weibel, M.A. and Majno, G. Am. J. Surg. 126: 345-353 (1973)).

The process of fibrous adhesion formation initially involves the establishment of a fibrin framework and normal tissue repair. The normal repair process allows for fibrinolysis alongside mesothelial repair. However, in fibrous adhesion formation the fibrin matrix matures as fibroblasts proliferate into the network and angiogenesis occurs resulting in the establishment of an organized fibrous adhesion within about 3 to 5 days (Buckman, R. F., et al., J. Surg. Res. 21: 67-76 (1976); Raferty, A. T., J. Anat. 129: 659-664 (1979)). Inflammatory processes include neutrophil activation in the traumatised tissues, fibrin deposition and bonding of adjacent tissues, macrophage invasion, fibroblast proliferation into the area, collagen deposition, angiogenesis and the establishment of permanent fibrous adhesion tissues.

Various attempts have been made to prevent surgical adhesions. These involve pharmacological approaches targeted at influencing the biochemical and cellular events that accompany surgical trauma as well as barrier methods for the separation of affected tissues. For example, the use of peritoneal lavage, heparinized solutions, procoagulants, modification of surgical techniques such as the use of microscopic or laparoscopic surgical techniques, the elimination of talc from surgical gloves, the use of smaller sutures and the use of physical barriers (films, gels or solutions) aiming to minimize apposition of serosal surfaces, have all been attempted. Currently, preventive therapies also include prevention of fibrin deposition, reduction of inflammation (steroidal and non-steroidal anti-inflammatory drugs) and removal of fibrin deposits.

Interventional attempts to prevent the formation of post-surgical adhesions have included the use of hydroflotation techniques or barrier devices. Hydroflotation involves the instillation of large volumes of polymer solutions such as dextran (Adhesion Study Group, Fertil. Steril. 40:612-619 (1983)), or carboxymethyl cellulose (Elkins, T. E., et al., Fertil. Steril. 41:926-928 (1984)), into the surgical space in an attempt to keep the organs apart. Synthetic barrier membranes made from oxidized regenerated cellulose (*e.g.,* Interceed™), polytetrafluoroethylene (Gore-tex surgical membrane) and fully resorbable membranes made from a modified hyaluronic acid/carboxymethylcellulose (HA/CMC) combination (Seprafilm™) have also been used to reduce post-surgical adhesion formation in both animals and humans (Burns, J. W., et al., Eur. J. Surg. Suppl. 577: 40-48 (1997); Burns, J. W., et al., Fertil. Steril. 66:814-821 (1996); Becker, J. M., et al., J. Am. Coll. Surg. 183:297-306 (1996)). The success of these HA/CMC membranes may derive from their ability to provide tissue separation during the peritoneal wound repair process when fibrous adhesions form. The membranes were observed to form a clear viscous coating on the injured tissue for 3-5 days after application, a time period that is compatible with the time course of post-surgical adhesion formation (Ellis, H., Br. J. Surg. 50: 10-16 (1963)). Unfortunately, limited success has been seen with these methods.

WO 03/018033 A1 relates to the use of fucans, such as fucoidan, in connection with a pharmaceutical agent and to the treatment of adhesion. According to this document, the fucan can be administered as a pharmaceutical composition comprising the fucan and also an amount of at least one other drug. For the treatment of adhesion, the fucan containing composition can be applied in form of suspensions, films, pastes, gels, sprays, liquids, lotions, implants and others.

Clearly there is an unmet need for compounds, compositions, methods and the like (including delivery approaches) to inhibit, or otherwise treat and/or prevent, the formation of fibrous adhesions, preferably more effectively with few side effects. The present compounds, compositions, methods, etc., provide one or more of these advantages.

### SUMMARY

The above defined problems are solved by the teaching of the present invention as defined by the independent main claim.

The present invention comprises agents comprising one or more of the anti-fibrous adhesion agents discussed herein, for the treatment of surgical adhesions. The anti-fibrous adhesion agents provide significant therapeutic effect against fibrous adhesions while typically also providing low side effects. Further, since a variety of different anti-fibrous adhesion agents are discussed, various combinations of the agents can be selected as desired to reduce side effects in a patient potentially suffering from other diseases or conditions, and/or to provide other beneficial healthful or therapeutic effects, such as compositions that both inhibit fibrous adhesions and also treat cancer or arthritis or swelling or any of the variety of other diseases or conditions that can also be treated by one or more of the anti-fibrous adhesion agents herein. The compositions disclosed herein are also useful for the treatment of fibrous growths and conditions such as keloid trait that share similar biology with fibrous adhesions. Accordingly, the discussion herein applies to such fibrous growths as well.

In one aspect, methods of inhibiting a fibrous adhesion in an animal comprising selecting an agent to inhibit the fibrous adhesion and administering a therapeutically effective amount of the agent to a site suspected of having the fibrous adhesion are dsiclosed. The agent can comprise one or more of an alginic acid, a doxycycline, a cortisone, an estramustine, a melezitose, a succinic acid, a meclofenamate, a palmitic acid, a dextran sulfate, collagen, a budesonide, an enalapril such as enalapril maleate, a nabumetone, a statin such as simvastatin, a captopril, a chitosan, a minocycline, a methotrexate, a cisplatin, an ibuprofen, an erythromycin, a tetracycline, an SDF-1 inhibitor such as an anti-SDF-1 antisense oligonucleotides (ASO), an anti-SDF-1 small molecule RNA, an anti-SDF-1 siRNA, an anti-SDF-1 ribozyme, an anti-SDF-1 aptamer, a small molecule inhibitor of SDF-1, an anti-SDF-1 antibody such as anti-hSDF-1/PBSF, a rapamycin, a hydroxypropylcellulose, a busulfan, a cyclophosphamide, a dacarbazine, a hydroxyurea, a mitotane, a docetaxel, a vinblastine sulfate, a MG132, a nimesulide, a diclofenac, a tenoxicam, an indomethacin, an acetylsalicylic acid, a diflusinal, a betamethasone, a dexamethasone, a deferoxamine mesylate, a retinoic acid, a heparin, a pentoxifylline, a streptokinase, a TGF-beta, a TIMP-2, a dextrose, a Dextran T70, a starch, a quercetin dihydrate, a caffeine, a leflunomide, a carrageenan such as iota-carrageenan or lambda-carrageenan, a hydroxypropylcellulose, a stachyose, a chondroitin sulfate A.

The agents can also be an anti-neoplastic agent, an anti-inflammatory agent, an iron-chelating agent, a triene macrolide antibiotic, a 3-hydroxy-3-methylgluteryl-CoA reductase inhibitor, a retinoid, an antithrombotic, an anticoagulant, a plasminogen activator, a cytokine, a matrix metalloproteinase inhibitor, a tetracycline, an ACE inhibitor, a dextran sugar, or a carrageenan, alkylating agent, an antimetabolite, a ribonucleotide reductase inhibitor, a cytotoxic antibiotic, a taxane, a vinca alkaloid, or a protease inhibitor, a COX-2 inhibitor, a fenamate, an oxicam, an acetyl acid derivative, a salicylic acid derivative, or a corticosteroid.

As noted elsewhere, the various aspects and embodiments disclosed herein can be features, etc., can be mixed and matched, combined and permuted in any desired manner. Thus, the particular agents above and sites and agents below, etc., can be combined, etc., as appropriate even if they do not appear together in the same paragraph.

In some embodiments, the subject or patient is an animal, such as a human, dog, cat, horse, cow, or other mammal, or bird, reptile or other animal. The treatment site can be a surgical site, a pelvic inflammatory disease site, a mechanical injury site, a radiation exposure site, a site suffering presence of a foreign material or any other desired site. The site can be the animal as a whole, or a specific site within an abdomen, limb, within a spine, a head, a reproductive tract, a gastrointestinal tract, a pulmonary system, thoracic cavity, cardiac or vascular system, a urinary system, or any other system or location as desired.

The drug can be substantially continuously administered to the disease site via controlled release from a polymeric dosage form. The administration form can comprise a film, patch, paste, microsphere, implant, gel, spray or liquid, solution, suspension, which can be in Lactated Ringers Injection USP. According to the invention, the agent is administered in combination with a fucan, which can be fucoidan. The agent can be administered in combination with a second agent, which can be any one or more of the other agents herein or any other therapeutic agent.

Further disclosed are pharmaceutical compositions configured to inhibit fibrous adhesions, the compositions comprising a therapeutically effective amount of a fucan selected to inhibit the fibrous adhesion, a therapeutically effective amount of at least one of the therapeutically effective agents herein selected to inhibit the fibrous adhesion, and at least one pharmaceutically acceptable excipient, carrier or diluent. The pharmaceutically acceptable excipient, carrier or diluent can if desired be selected from the group consisting of a pluronic, cellulose, alginate, acrylate, hyaluronic acid, polyethylene glycol, and chitosan.

The compositions can be used in the manufacture of a medicament for treating a fibrous adhesion, and can be used in methods of manufacturing a medicament able to reduce symptoms associated with a fibrous adhesion in a human patient, for example comprising combining a pharmaceutically effective amount of fucoidan, a therapeutically effective amount of at least one of the therapeutically effective agents herein selected to inhibit the fibrous adhesion, and a pharmaceutically acceptable excipient or buffer.

In still other aspect, methods of treating at least one of a non-fibrous adhesion disease or non-fibrous adhesion condition in an animal are disclosed. The methods can comprise identifying the non-fibrous adhesion disease or condition, and comprise selecting at least one therapeutic agent for the non-fibrous adhesion disease or condition, selecting at least one anti-fibrous adhesion agent, and administering at least one pharmaceutical composition comprising a therapeutic amount of the at least one therapeutic agent for the non-fibrous adhesion disease or condition and a therapeutic amount of the at least one anti-fibrous adhesion agent.

The at least one therapeutic agent for the non-fibrous adhesion disease or condition and the therapeutic amount of the at least one anti-fibrous adhesion agent can be in at least two different compositions and the methods further can comprise administering the compositions substantially simultaneously. The agents can also all be in a single composition. The agents can be administered to the site via controlled release from a polymeric dosage form, as a solution or suspension, or otherwise as desired.

In still another aspect, pharmaceutical compositions are disclosed, which are configured to treat at least one of a non-fibrous adhesion disease or non-fibrous adhesion condition in an animal, and to inhibit fibrous adhesions, the compositions comprising a therapeutically effective amount of at least one therapeutic agent for the non-fibrous adhesion disease or condition selected to treat the non-fibrous adhesion disease or condition, a therapeutically effective amount of at least one anti-fibrous adhesion agent selected to inhibit the fibrous adhesion, and at least one pharmaceutically acceptable excipient, carrier or diluent.

The compositions can be used in the manufacture of a medicament for treating at least one of a non-fibrous adhesion disease or non-fibrous adhesion condition and for inhibiting a fibrous adhesion in an animal.

Further disclosed are methods of manufacturing a medicament able to reduce symptoms associated at least one of a non-fibrous adhesion disease or non-fibrous adhesion condition, and also inhibit symptoms associated with a fibrous adhesion, in a human patient, comprising combining therapeutically effective amount of at least one therapeutic agent for the non-fibrous adhesion disease or condition selected to treat the non-fibrous adhesion disease or condition, a therapeutically effective amount of at least one anti-fibrous adhesion agent selected to inhibit the fibrous adhesion, and at least one pharmaceutically acceptable excipient, carrier or diluent.

In still yet a further aspect, methods of inhibiting a fibrous adhesion in an animal are disclosed, comprising selecting an agent to inhibit the fibrous adhesion and administering a pharmaceutical compositions comprising a therapeutically effective amount of the agent to a site suspected of having the fibrous adhesion, wherein the compositions can be configured to inhibit at least a certain portion of fibrous adhesions, for example about 75%, 90%, 99%, or substantially all of the fibrous adhesions. The efficacy can be determined via any desired standard, for example relative to hyaluronic acid film without any anti-fibrous adhesion agent, which can be used for example in a human, rat or rabbit model. The embodiments also include pharmaceutical compositions configured to inhibit a fibrous adhesion in an animal comprising a selected anti-fibrous adhesion agent, wherein the compositions can be configured to inhibit at least a certain portion of fibrous adhesions, for example about 75%, 90%, 99%, or substantially all of the fibrous adhesions.

In still yet another further aspect, kits are disclosed. The kits can comprise a vessel containing the compositions herein and a label comprising instructions for pharmaceutical use of the compositions to inhibit fibrous adhesions. The label can be a government approved label such as an FDA approved label. The vessel can be a vial configured to hold an instillate or any other desired composition form herein. The label further can comprise instructions for pharmaceutical use of the compositions to treat at least one of a non-fibrous adhesion disease or non-fibrous adhesion condition.

These and other aspects, features and embodiments are set forth within this application, including the following Detailed Description and attached drawings. In addition, various references are set forth herein, including in the Cross-Reference To Related Applications, that discuss certain systems, apparatus, methods and other information.

### BRIEF DESCRIPTION OF THE FIGURES

Figure 1 is a graph depicting the results of an anti-SDF-1 on the inhibition of fibrous adhesions using the rat caecal-sidewall adhesion model.
Figure 2 is a graph depicting the results of rapamycin on the inhibition of fibrous adhesions using the rat caecal-sidewall adhesion model.
Figure 3 is a graph depicting the results of various anti-neoplastic agents on the inhibition of fibrous adhesions using the rat caecal-sidewall adhesion model.
Figure 4 is a graph depicting the results of various anti-inflammatory agents on the inhibition of fibrous adhesions using the rat caecal-sidewall adhesion model.
Figure 5 is a graph depicting the results of various agents on the inhibition of fibrous adhesions using the rat caecal-sidewall adhesion model.
Figure 6 is a graph depicting the results of fucoidan film or fucoidan instillate formulations on the inhibition of fibrous adhesions using the rat caecal-sidewall adhesion model.
Figure 7 is a graph depicting the results of series of fucoidan gel formulations on the inhibition of fibrous adhesions using the rat caecal-sidewall adhesion model.
Figure 8 is a graph depicting the results using a 0.001%, 0.003%, and 0.01% w/v fucoidan instillate formulations on the inhibition of fibrous adhesions using the rat caecal-sidewall adhesion model.
Figure 9 is a graph depicting the results using 3% and 0.3% w/v fucoidan instillate formulations on the inhibition of fibrous adhesions using the rabbit uterine horn model.
Figure 10 is a graph depicting the results of 0.001% w/v fucoidan instillates produced from both *Fucus vesiculosis* and *Laminaria japonica* (Kombu) on the inhibition of fibrous adhesions using the rat caecal-sidewall adhesion model.

### DETAILED DESCRIPTION

In some embodiments, the present invention uses the agents discussed herein to inhibit, e.g., treat or prevent, the formation of fibrous adhesions, which may form following surgery, following trauma, or following radiation or chemotherapy, or as a result of any other cause, by application of the agent(s) to the tissue of an animal, including a human, dog, cat, horse, cow, or other mammal, or bird, reptile or other animal at site suspected of developing a fibrous adhesion, for example sites actually having a fibrous adhesion, sites unduly subject to developing a fibrous adhesion, for example due to exposure to radiation, surgery, disease, or injury, and sites in the process of developing or expanding fibrous adhesions. Each agent listed includes the agent and all its derivatives, salts, and analogues without exclusion unless expressly stated otherwise. The agents can be administered in different formulations for the inhibition of fibrous adhesions. These compositions can if desired allow for release of effective doses of the agents at the disease sites only, in order to reduce toxicity that may be associated with systemic delivery of some of these compounds. These compositions can also comprise polymeric formulations of an agent herein (including all derivatives, salts and analogues thereof), or other formulations as desired, which can provide sustained release of the agent at the potential fibrous adhesion site. The compositions, methods, etc., discussed herein include formulations comprising each agent discussed herein, whether it be used alone, or in conjunction with fucoidan (or any other fucan), or in conjunction with any other agent discussed herein, or any other agent, device, or barrier, or with any combination of drugs including fucoidan, and the agents discussed herein, and any other agent. The compositions can be administered to a site directly, systemically or otherwise as desired. In certain embodiments, the compositions herein do not include any antisense oligonucleotides or other oligonucleotide agents such as gene therapy nucleotides.

In some exemplary embodiments disclosed herein, the methods and compositions relate to the use of just one of the various anti-fibrous adhesion agents herein, or to the use of two or more of such agents. In some embodiments, at least one of the agents in such compositions, including both solo and multiple agent mixture compositions, is a fucan.

The compositions herein are also useful for the treatment of fibrous growths and conditions such as keloid trait that share similar biology with fibrous adhesions. Accordingly, the discussion herein applies to such fibrous growths as well.

The exemplary embodiments disclosed herein can include identifying a non-fibrous adhesion disease or condition, then selecting and administering a composition comprising an anti-fibrous adhesion agent that also or simultaneously treats or inhibits both the non-fibrous adhesion disease or condition and the fibrous adhesion. In some exemplary embodiments, the compositions and methods can further comprise selecting two or more of the agents herein, such that one has primary effect against the non-fibrous adhesion disease or condition and the other has primary effect against the fibrous adhesion. Further, the compositions and methods can comprise identifying, selecting and administering at least one anti-fibrous adhesion agent such as those discussed herein and at least one agent against the non-fibrous adhesion disease or condition, administered together in a single or simultaneous compositions. Thus, the methods can comprise selecting an agent to inhibit the fibrous adhesion and selecting the same or at least one other agent to inhibit the non-fibrous adhesion disease or condition, and administering a therapeutically effective amount of the agent(s) to a site suspected of developing the non-fibrous adhesion disease or condition and the fibrous adhesion. Exemplary non-fibrous adhesion diseases or conditions include cancers, PID, radiation exposures, mechanical and other injuries, arthritis, psoriasis, surgery, topical conditions, diseases and conditions of the GI tract, for example those that have substantial risk of blockages or other mechanically disruptive symptoms, etc.

Within certain embodiments of the invention, the anti-fibrous adhesion agents may be formulated along with other compounds or compositions, such as, for example, an ointment, solution, cream, lotion, gel, spray, mousse, coating, wrap, paste, barrier, implant, microsphere, microparticle, film, particulate, liquid, implant films, instillate formulations and the like.

Generally, compositions disclosed herein can be administered alone or as part of a composition by application or injection as a paste, gel, spray, particulate, film, solution, liquid, lotion, cream or implant. Routes and sites of administration include orally, systemically, intraocularly, subcutaneously, intraperitoneally, intramuscularly, intraarticularly, intralesionally, intravaginally, rectally or topically, such as in a patch.

The therapeutically effective amount of the agent can comprise about 0.1%, 0.5%, 1%, 5% to 50%, 20-80%, 80% to 100% w/v or w/w as desired of the composition. The compositions herein can be provided in suitable vessels or containers, which in turn can be provided in kits and can also be provided with a label, preferably a label approved by an appropriate government regulatory agency such as the Food and Drug Administration in the United States of America. The label can comprising instructions for pharmaceutical use of the composition. The vessel can be, for example, a vial, and can be configured to provide the composition(s) as films, gels, instillates, or other forms discussed herein or as other wise desired.

The compound or composition given with the anti-fibrous adhesion agents may function as a carrier and/or as a physical barrier, which may be either polymeric or non-polymeric. The compositions discussed herein also comprise agents (or any combination of agents from the list of agents discussed herein including fucoidan or other fucan) alone or in aqueous solution, or non-aqueous solution, or dispersed as a suspension within a vehicle or carrier. Representative examples of polymeric carriers, barriers and excipients include chitosan, polytetrafluoroethylene, poly(lactic acid), poly-(ethylene vinyl acetate), poly(glycolic acid), copolymers of ethylene and vinyl acetate, polyethylene glycol, methoxypolyethylene glycol, polycaprolactone, copolymers of lactic acid and glycolic acid, copolymers of poly(lactic acid) and poly(caprolactone), gelatin, collagen, celluloses, albumen, pluronics, poly-(valerolactone), poly-(anhydrides), polysaccharides, alginic acids such as alginates, hyaluronic acid, injectable excipients other polymeric based vehicles and copolymers, derivatives mixtures and blends thereof. Representative examples of other suitable carriers include ethanol, glycols including ethylene glycol, propylene glycol or Transcutol®, mixtures of ethanol and glycols, isopropyl myristate or isopropyl palmitate, mixtures of ethanol and isopropyl myristate or isopropyl palmitate. Such polymers may, themselves, provide anti-adhesion activity in certain compositions.

### GENERAL DISCUSSION OF EXEMPLARY ANTI-FIBROUS ADHESION AGENTS

The drug components of the compositions herein typically are well known for other compositions and purposes. The following provides some information about some of them.

NSAIDs. The major mechanism by which the NSAIDs elicit their therapeutic effects (antipyretic, analgesic, and anti-inflammatory activities) is inhibition of prostaglandin (PG) synthesis. Specifically NSAIDs competitively (for the most part) inhibit cyclooxygenases (COXs), the enzymes that catalyze the synthesis of cyclic endoperoxides from arachidonic acid to form prostaglandins). Other mechanisms that may contribute to NSAID anti-inflammatory activity include the reduction of superoxide radicals, induction of apoptosis, inhibition of adhesion molecule expression, decrease of nitric oxide synthase, decrease of proinflammatory cytokine levels (tumor necrosis factor-a, interleukin-1), modification of lymphocyte activity, and alteration of cellular membrane functions.

COX-2 inhibitors. (Int. J. Immunopathol. Pharmacol. 2003 May-Aug;16(2 Suppl):17-22).

Their action is centered on the inhibition of the cyclooxygenase (COX) enzyme responsible for converting arachidonic acid to prostaglandins and throboxane. In 1991, it was disclosed that COX exists in two distinct isozymes (COX-1 and COX-2), one of which, COX-2, is primarily responsible for inflammation but apparently not for gastrointestinal integrity or platelet aggregation. For this reason, in recent years, novel compounds that are selective for this isozyme, the so-called selective COX-2 inhibitors or COXIBs, which retain anti-inflammatory activity but minimize the risk of gastrointestinal toxicity and bleeding, have been developed. Some of the COX-independent mechanisms of COX-2 inhibitors include activation of protein kinase G, inhibition of NF-kappa B activation, downregulation of the antiapoptotic protein Bcl-XL, inhibition of PPAR delta, and activation of PPAR gamma.

COX-2 inhibitors include:
Nimesulide (CAS 51803-78-2) (Drugs. 2003;63 Suppl 1:9-22.)
Fenamates. (Prim Care. 1990 Sep;17(3):589-601)

These agents are considered to be N-aryl substituted derivatives of anthranilic acid which is itself a bioisostere of salicylic acid. These agents retain the acidic properties that are characteristic of this class of agents. The most active fenamates have small alkyl or halogen substituents at the 2',3' and/or 6' position of the N-aryl moiety mefenamate- see below). Among the disubstituted N-aryl fenamates the 2',3'-derivatives are most active suggesting that the substituents at the 2',3'-positions serve to force the N-aryl ring out of coplanarity with the anthranilic acid. Hence this steric effect is proposed to be important in the effective interaction of the fenamates at their inhibitory site on cyclooxygenase. Actions: The anthranilates have primarily antiinflammatory with some analgesic and antipyretic activity and are non-COX selective. The anthranilates are used as mild analgesics and occasionally to treat inflammatory diseases.

Fenamates include:
Meclofenamic acid - (CAS 644-62-2)
Meclofenamate (CAS 6385-02-0)
Diclofenac - (CAS 15307-86-5) derived from 2-arylacetic acid, used for RA, OA, AS and post-op pain,
Oxicams. (Arthritis Rheum. 1997 Jan;40(1):143-53).

Oxicams are characterized by the 4-hydroxybenzothiazine heterocycle. The acidity of the oxicams is attributed to the 4-OH with the enolate anion being stabilized by intramolecular H-bonding to the amide N-H group. Also, the presence of the carboxamide substituent at the 3-position of the benzothiazine ring contributes toward acidity by stabilizing the negative charge formed during ionization (resonance stabilization). Although these compounds are acidic (pKa = 6.3), they are somewhat less acidic than carboxylic acids NSAIDs. Yet the oxicams are primarily ionized at physiologic pH and acidity is required for COX inhibitory activity.

Oxicams include:
Tenoxicam (CAS 59804-37-4)
Acetyl acid derivatives. (FASEB J. 2001 Oct;15(12):2057-72).

These compounds are also derivatives of acetic acid, with the substituent at the 2-position being a heterocycle or related carbon cycle.

Acetyl acid derivatives include:
Indomethacins (CAS NO. 53-86-1) (Indocid, Intodec) - indole-3-acetic acid derivatives containing a benzoylated indole nitrogen. The methyl group at the 2 position of the indole ring prevents free rotation about the C-N bond and keeps the two aromatic rings in the correct relationship for COX binding and therapeutic activity. Indomethacin is "COX-1" selective" and produces primarily antiinflammatory actions with some analgesic and antipyretic activity.

Salicylic acid derivatives. Structure and Chemistry: The salicylates are derivatives of 2-hydroxybenzoic acid (salicylic acid). The salicylates were discovered in 1838 following the extraction of salicylic acid from willow bark. Salicylic acid was used medicinally as the sodium salt but replaced therapeutically in the late 1800s by the acetylated derivative, acetylsalicylic acid (ASA) or aspirin. Therapeutic utility is enhanced by esterification of the phenolic hydroxyl group as in aspirin, and by substitution of a hydrophobic/lipophilic group at C-5 as in diflunisal. The salicylates have potent antiinflammatory activity with mild analgesic and antipyretic activities. These compounds are mainly "COX-1 selective" - they are bound with higher affinity by COX-1. Toxicities include GI irritation, hypersensitivity reactions, inhibition of platelet aggregation, and ototoxicity (tinnitus). The therapeutic and certain of the toxic actions (*i.e.,* gut) of aspirin can be related to its ability to inhibit COX in various tissues and participate in transacetylation reactions *in vitro.* For example, acetylation of COX results in irreversible inhibition of this enzyme and antiinflammatory effects in joints, and adverse effects in the GI tract. Also acetylation of circulating proteins may result in a hypersensitivity response.

Salicylic acid derivatives include:
Acetylsalicylic acid (CAS Number 50-78-2)
Diflunisal (CAS 22494-42-4) - the difluorophenyl analogue of salicylic acid differs from other members of the salicylate class in that it has primarily analgesic and antipyretic activity. It is used to treat the pain associated with RA, OA and muscle pain. It reported causes less GI tract ulceration than aspirin and has lower auditory side effects. This drug is cleared primarily by phenol and carboxyl O-glucuronidation similar to the salicylates.

Pyrazalones. This class of agents is characterized by the 1-aryl-3,5-pyrazolidinedione structure and are structurally related to the aromatic compound pyrazole These compounds are analgesic, antipyretic, anti-inflammatory (due to their weak acidity) and uricosuric at near toxic doses. The acidity in these molecules is due to the presence of an enolizable hydrogen in the 4 position, and is pKa-dependent.

Pyrazalones include:
Phenylbutazone (CAS 50-33-9)

Corticosteroids. Corticosteroids are a group of anti-inflammatory drugs similar to the natural corticosteroid hormones produced by the cortex of the adrenal glands. Among the disorders that often improve with corticosteroid treatment are asthma, allergic rhinitis allergic, eczema and rheumatoid arthritis. How these anti-inflammatory agents inhibit late phase allergic reactions occurs via a variety of mechanisms, including decreasing the density of mast cells along mucosal surfaces, decreasing chemotaxis and activation of eosinophils, decreasing cytokine production by lymphocytes, monocytes, mast cells and eosinophils, inhibiting the metabolism of arachidonic acid and other mechanisms.

Corticosteroids include:
Dexamethasone (CAS 50-02-2)

Alkylating agents. An alkylating agent is a compound that substitutes an alkyl group, Cn H 2n+1, for an active hydrogen atom in an organic compound, with DNA as the principal target. Alkylating agents were developed from mustard gas in 1946. Reaction with DNA, RNA or proteins leads to alkylation, which may be bifunctional and cause DNA crosslinking groups include nitrogen mustards, nitrosoureas, and platinum containing drugs as well as others. All of the alkylating agents form strong electrophiles through the formation of carbonium ion intermediates. This results in the formation of covalent linkages by alykylation of various nucleophiles moieties. The chemotherapeutic and cytotoxic effects are directly related to the alkylation of DNA mainly through the 7 nitrogen atom of guanine although other moieties are also alkylated. The formation of one covalent bond with nucleophiles can result in mutagenesis or teratogenesis but the formation of two of these bonds through cross linking can produce cytotoxicity.

Examples of alkylating agents include:
Busulfan (CAS 55-58-1) (Busulfex, Myleran)
cyclophosphamide (CAS 6055-19-2) (Procytox)
estramustine (CAS:2998-57-4) (Emcyt)
cisplatin (CAS 15663-27-1)
dacarbazine (CAS 4342-03-4)

Antimetabolites. (Semin Oncol. 1992 Dec;19(6):695-706).

An antimetabolite is defined as a compound that interferes with the utilization of a natural metabolite by means of having a similar chemical structure. Antimetabolites are generally analogues of steroid hormones or nucleic acid precursors. Nucleic acid and folate antimetabolites act by inhibition of DNA and/or RNA synthesis. Their mode of action therefore means that their toxic effects are most marked in rapidly proliferating tissues. There are several different cellular targets for antimetabolites. Some common classes of antimetabolites are: folate antagonists, purine antagonists and pyrimidine antagonists.

Examples of antimetabolite agents include:
Methotrexate (CAS 59-05-2)

Ribonucleotide reductase inhibitors. Ribonucleotide reductase inhibitors may bind with the R1 subunit of the enzyme ribonucleotide reductase which catalyzes the de novo biosynthesis of deoxyribonucleosides therefore interfering with DNA synthesis. (Expert Rev Anticancer Ther. 2002 Aug;2(4):437-48).

Examples of ribonucleotide reductase inhibitors include:
Hydroxyurea (CAS 127-07-1) (Hydrea)

### Cytotoxic antibiotics.

Examples of cytotoxic antibiotics include:
Mitotane (CAS 53-19-0)

Taxanes. Taxanes block cell cycle progression by stabilizing microtubules resulting in centrosomal impairment, induction of abnormal spindles and suppression of spindle microtubule dynamics (Curr Cancer Drug Targets. 2003 Jun;3(3):193-203).

Examples of topoisomerase inhibitors include:
Docetaxel (CAS 114977-28-5) (Taxotere)

Vinca alkaloids and analogues. (Curr Med Chem Anti-Canc Agents. 2002 Jan;2(1):1-17).

Vinca alkaloids inhibit microtubule polymerization by binding to sites on tubulin and therefore block mitosis at the metaphase/anaphase transition, and induce cell death.

Examples of vinca alkaloids include:
Vinblastine (CAS 865-21-4)

### Proteasome inhibitors. (Cancer Treat Rev. 2003 May;29 Suppl 1:41-8)

The proteasome is the final degradative enzyme involved in an important catabolic pathway for many intracellular regulatory proteins including IkB/NF-kB, p53, and the cyclin-dependent kinase inhibitors p21 and p27. The antineoplastic effect of proteasome inhibitors may involve several distinct mechanisms including inhibition of cell growth signaling pathways, induction of apoptosis, and inhibition of cellular adhesion molecule expression.

Examples of proteasome inhibitors include:
MG132 (Cytokine. 2003 Nov 7;24(3):67-73), inhibits NF-kappaB formation and degradation of its inhibitor I-kappaB.

Iron-Chelating Agents. (Adv Exp Med Biol. 2002;509:231-49). Orally active iron-chelating drugs, used therapeutically in conditions of transfusional iron overload and for the treatment of iron overload in thalassaemia.

Examples of iron-chelating agents include:
Deferoxamine mesylate (CAS 138-14-7) - Binds to free iron, iron of ferritin, and hemosiderin forming ferrioxamine, which is a water-soluble chelate excreted by the kidneys (urine is a reddish color) as well as in the feces via the bile. Rapidly metabolized by plasma enzymes and excreted in the urine.

3-Hydroxy-3-Methylgluteryl-CoA Reductase Inhibitors. These drugs inhibit 3-hydroxy-3- methylglutatyl-coenzyme A -CoA reductase catalyzes the conversion of HMG-CoA to mevalonate, which is an early and rate-limiting step in the biosynthesis of cholesterol.

Examples of 3-Hydroxy-3-Methylgluteryl-CoA Reductase Inhibitors include:
Statins
Simvastatin (Zocor) (CAS 79902-63-9).

Retinoids and retinoid analogues. (J Dermatol. 2003 May;30(5):355-80.).

Retinoids (natural and synthetic derivatives of vitamin A) signal potent differentiation and growth-suppressive effects in diverse normal, premalignant, and malignant cells. Retinoids include all- trans-retinoic acid (ATRA), a major active form of vitamin A (retinol), and its bioisosters, which elicit their biological effects by binding to their nuclear receptors, retinoic acid receptors (RARs).

Examples of Retinoids and retinoid analogues include:
All-trans-retinoic acid (CAS 302-79-4) J Biol Regul Homeost Agents. 2003 Jan-Mar;17(1):98-114).

Antithrombotics. Drugs which interact with thrombin and block its catalytic activity on fibrinogen, platelets and other substrates. (Expert Opin Pharmacother. 2003 May;4(5):653-66).

Examples of antithrombotics include:
Heparin sodium (CAS 9041-08-1).

Low molecular weight heparins (Semin Thromb Hemost. 2000;26 Suppl 1:31-8). As compared with the standard heparin, LMWHs have different pharmacodynamic, and pharmacokinetic properties; they also differ in clinical benefits. LMWHs have greater bioavailability, longer half-lives, a more predictable pharmacologic response, possible improved safety, and similar or greater efficacy compared with unfractionated heparin.

### Anticoagulants.

Examples of anticoagulants include:
Pentoxifylline (CAS 6493-05-6).

### Plasminogen Activators.

Examples of plasminogen activators include:
Streptokinase (CAS 9002-01-1).

### Cytokines.

Examples of cytokines include:
Transforming Growth Factor -Beta (TGF-β, J Biol Chem. 2002 Aug 30;277(35):31938-48).

Matrix Metalloproteinase Inhibitors. (Hematol Oncol Clin North Am. 2002 Oct;16(5):1189-227).

Tissue inhibitors of matrix metalloproteinases (TIMPs) have been shown to block tumor cell invasion suggesting that they act as metastasis suppressor genes. Their primary function ins to inhibit matrix metalloproteinases (MMPs) which are Zn(2+)-binding endopeptidases that degrade various components of the ECM. MMPs are enzymes implicated in normal and pathologic tissue remodeling processes, wound healing, angiogenesis, and tumor invasion.

Examples of matrix metalloproteinase inhibitors include:
TIMP-2.

Tetracyclines. The tetracyclines are closely congeneric derivatives of the polycyclic napthacenecarboxamide. The tetracyclines possess a wide range of antimicrobial activity against gram-positive and gram-negative bacteria. In vitro, these drugs are primarily bacteriostatic. The tetracyclines and their non-antimicrobial, chemically modified analogues have properties that appear to modulate host response by inhibiting the activity of the matrix metalloproteinases that cause collagen destruction. They also inhibit osteoclast function, stimulate osteoblastic bone formation, and regulate angiogenesis.

Examples of tetracyclines include:
Tetracycline (CAS 60-54-8).
Minocycline (CAS 10118-90-8).
Doxycycline (CAS 564-25-0).

Angiotensin-Converting Enzyme (ACE) Inhibitors. ACE inhibitors act basically as inhibitors of the renin-angiotensin vasoconstrictor system, and are used to treat hypertension and congestive heart failure. They have also been shown to reduce proinflammatory mediators, such as interleukin-6, and enhance the concentration of anti-inflammatory cytokines, such as interleukin-10.

Examples of Angiotensin-Converting Enzyme Inhibitors include:
Captopril (CAS 62571-86-2).
Enalaprils including salts thereof such as enalapril maleate (*e.g.,* 5% w/w) (CAS 76095-16-4)

### Miscellaneous.

Examples of certain other desired agents include:
leflunomide (Arava) - an isoxazole immunomodulator that interferes with the metabolism of pyrimidine by inhibiting dihydro-orotate dehydrogenase (DHO-DH) in mitochondria, thereby blocking T- and B cell proliferation. (Expert Opin. Pharmacother. 2003 Jun;4(6):987-97.).
Erythromycin.
Dextran sulfate.
Alginic acid.
Dextrose.
Dextran T70.
Starch.
Quercetin Dihydrate.
Caffeine.
?-Carrageenan.
?-Carrageenan.
Hydroxypropylcellulose.
Stachyose.
Chondroitin Sulfate A.

### FUCANS

Fucans (including fucoidan) are high molecular weight sulphated polysaccharides extracted from brown seaweeds, Percival, E., and McDowell, R. H., Chemistry and Enzymology of Marine Algal Polysaccharides, pp. 157-175 (Academic Press, New York, 1967), and as is well known can be found from other sources as well, Vasseur, E., Chemical studies on the jelly coat of the sea-urchin egg. Acta Chem. Scand., 2, 900-913 (1948); Mourao, PAS and Bastos, IG, Highly acidic glycans from sea cucumbers. Eur. J. Biochem., 166, 639-645 (1987); Pereira, et. al., Structure and Anticoagulant Activity of Sulfated Fucans, J. Biol. Chem., 274:12. 7656-7667 (1999). Fucoidan (or fucoidin) indicates fucans derived from brown seaweed. USPA 2003064958. Fucans can be alone, or in a mixture, for example in a mixture of sugars such as xylose, galactose, glucose and/or mannose. These sugars are known to be contained in the marine algae and are may be extracted with the fucan. Duarte, Maria ER., Cardoso, Marc A., Noseda, Miguel D., Cerezo, Alberto S., "Structural studies on fucoidans from the brown seaweed Sargassum stenophyllum". Carbohydrate Research: 2001 (333): 281-293

These compounds reportedly have multiple inhibitory actions *in vivo* and *in vitro* including anti-thrombin, anti-proliferative, anti-complement, anti-cancer and anti-neutrophil migration effects. Fucans may block various binding events at cell surfaces including cell-cell binding through integrin-selectin molecules, or by binding thrombin or complement in the blood or fucose receptors on cell surfaces.

Fucans have been shown to have anticoagulant effects and that this anticoagulant activity is related to the density of sulphate groups (Pereira, M.S. et al., J Biol Chem. 274(12): 7656-7667 (1999).

Such activity is thought to be responsible for anti-inflammatory properties via (for example) inhibition of lymphocyte or neutrophil binding to vascular endothelial cells that might prevent the invasion of these cells into a tissue compartment with subsequent inflammation (Patankar, M.S., et al., J. Biol. Chem. 268: 21770-21776 (1993); Brandley, B.K., et al., J. Cell Biol. 105: 991-997 (1987)). Recent studies have also shown that fucans inhibit vascular smooth muscle cell proliferation (Logeart, D., et al., Eur. J. Cell Biol. 74: 376-384 & 385-390 (1997)), indicating (but not demonstrating) a possible anti-restenosis potential of these compounds. Fucans have been shown to be slowly internalized in cells following surface binding to both endothelial and smooth muscle cells (Glabe, C.G., et al., J. Cell Science 61: 475-490 (1983); Logeart, D., et al., Eur. J. Cell Biol. 74: 376-384 (1997)).

In Japan, fucoidan extracted from various seaweeds is marketed as a health food (Riou, D., et al., Anticancer Res., 16 (3A): 1213-1218 (1996); Itoh, H., Anticancer Res., 13 (6A): 2045-2052 (1993); Nishiro, T., et al., Thromb. Res., 62: 765-773 (1991); Blondin, C., et al., Mol. Immunol., 31: 247-253 (1994); Patankar, M.S., et al., J. Biol. Chem., 268: 21770-21776 (1993)). Fucoidan has been proposed as a cosmetic or dermal agent. JP 01031707 and JP 01085905. Fucoidan has been reported to be a potential anticancer agent (Riou. D., Anticancer Res. 16: 3a 1213-18 (1996); Itoh, H., et al., Anticancer Res., 15: 5b 1937-47 (1995)). Fucoidan was reported to not inhibit angiogenesis *in vitro* (Soeda, S., et al., Biochim. Biophysica Acta (1): 127-134 (2000)). Similarly, fucoidan was found to stimulate HUVEcell proliferation (*in vitro*) induced by serum, indicating a possible proangiogenic effect (although inhibition was possible when fibroblast growth factor was present) (Giraux, J., et al., Eur. J. Cell Biol. 77 4: 352-9 (1998)). Studies have also shown that Fucans inhibit endothelial cell monolayer binding (Glabe, C.G., J. Cell Science, 61: 475-490 (1983)). Since the cells that make up capillaries are endothelial cells, this report indicates that *in vitro,* some aspects of cell adhesion may be inhibited but these data do not demonstrate any *in vivo* antiangiogenic effect of fucoidan. Fucoidan has been reported to inhibit the binding of *helicobacter* to gastric cells hinting at an antigastric ulcer effect (Shibat, H. J., Nutr. Sci. Vitaminol. 45: 325-336 (1999)).

Other sulphated fucans including linear, branched and linear sulphated fucans are reported to have differential anticoagulant activity (Pereira, M.S., J. Biol. Chem. 12: 7656-67 (1999)). Dextran sulphate and derivatives have been reported to inhibit cancer cell growth (Bittoun, P., Carbohydrate Res. (3-4): 247-255 (1999)) and to have anticoagulant effects (Mauray, S., J. Biomat. Sci. Poly ed. 9: 373-87 (1998)). Sulphated polysaccharides have been proposed as anti-viral agents for use against e.g., AIDS. EP 00293826; JP 01313433.

Fucans such as fucoidan can be obtained from a variety of species of brown algae including but not limited to: *Adenocystis utricularis, Ascophyllum nodosum, Chorda filum, Cladosiphon okamuranus, Cystoseira abies marina, Ecklonia kurome, Fucus evanescens, Fucus vesiculosis, Hizikia fusiforme, Kjellmaniella crassifolia, Laminaria brasiliensis, Laminaria cichorioides, Laminaria japonica* (commonly called Kombu) *Laminaria saccharina, Pelvetia fastigiata, Sargassum stenophylum, Sargassum thunbergii,* and *Undaria pinnatifida.* These species are all from the taxonomic class *Phaeophyceae* and the majority of these species fall into the families of *Fucales* and *Laminariaceae.*

Fucans suitable for this invention include those obtained from any source listed herein, as well as any additional sources in the taxonomic families of *Fucales* and *Laminariaceae,* or from other marine algae and seaweeds and echinoderms, sea cucumbers, sea urchins or other sources as desired including synthetic sources.

### FILMS

The agents discussed herein can be formulated as a film suitable for direct application to tissue of an animal, including a human, for the treatment of fibrous adhesions. The desired properties of the film include that it is thin, flexible, has the ability to be handled and is able to be affixed to tissue. Each agent discussed herein can also be incorporated into a polymer to create a film. The properties of the polymeric film formulation can be enhanced with the addition of suitable excipients. In one embodiment, the agent can be combined with hyaluronic acid polymer to make a film. Excipients which can be added include 1-ethyl-3-[3-(dimethylamino)propyl]carbodiimide (EDAC) and glycerol.

An embodiment of this invention is the incorporation of the agent to produce a 0.001% - 99% w/w drug (agent) loaded film. A second embodiment is the incorporation of the agent to produce a 50% - 99% w/w drug loaded film. A third embodiment is the incorporation of the agent to produce a 0.001 % - 50% w/w drug loaded film. A fourth embodiment is the incorporation of the agent to produce a 10% - 50% w/w drug loaded film. A fifth embodiment is the incorporation of the agent to produce a 30% - 40% w/w drug loaded film. A sixth embodiment is the incorporation of the agent to produce a 0.001% - 10% w/w drug loaded film. A seventh embodiment is the incorporation of the agent to produce a 1% - 10% w/w drug loaded film. An eighth embodiment is the incorporation of the agent to produce a 0.001% -1% w/w drug loaded film. A ninth embodiment is the incorporation of the agent to produce a 1% - 5% w/w drug loaded film. A tenth embodiment is the incorporation of the agent to produce a 1% - 2% w/w drug loaded film, or other concentrations discussed herein. One embodiment comprises the incorporation of the agent with hyaluronic acid yielding a 5% w/w drug loaded film, with the remainder of the film being made up of Hyaluronic acid, glycerol, and EDAC in approximately a 45:19:3 ratio.

### GELS

Each agent discussed herein can be incorporated into a viscous solution, which herein will be referred to as a gel. This gel can be administered to a body cavity of an animal, including a human, and is efficacious for the inhibition or prevention of fibrous adhesion formation.

Desired properties of the gel include that it is viscous enough to be applied to a specific location and remain affixed there, thus it will not flow under its own weight; and that it can be administered to the preferred location with the use of a syringe or injected through a needle. In one embodiment of the invention the viscous liquid was made using a 5.5% w/v hyaluronic acid solution. The agents discussed herein may be incorporated to yield a 0.001% - 1% w/v gel. The agent may also be integrated to produce a 1% - 10% w/v gel. The agent may also be loaded to produce a 10% - 50% w/v gel, or other concentrations discussed herein.

### INSTILLATES

Each agent discussed herein can also be dissolved or suspended in a liquid, which can be administered into a body cavity of an animal, including a human, and used to inhibit, treat, prevent, etc., the formation, including the increased growth, of fibrous adhesions. These formulations are herein referred to as instillate formulations. These formulations can, for example, be administered intra-abdominally following a surgical procedure into a patient to prevent the formation of post-operative adhesions, or into/onto any other desired wound, disease, etc., site. This liquid can be a solvent and can subsequently produce a solution of the agent. Additionally, the solvent used to dissolve the agent may be water-based. Dissolving the agent in an electrolytic solution can make the instillate formulation. The instillate is then administered to a suitable body cavity where it will prevent the formation of fibrous adhesions.

In some embodiments the instillate solution is a substantially non-viscous liquid, for example having a viscosity substantially similar to water, capable of reaching substantially all areas of a specific body cavity where it is introduced. The desired mixture may incorporate at least one agent discussed herein into a liquid to produce a solution (or suspension, etc.) at concentrations of between about 0.0001% w/v and 1% w/v, between 1% w/v and 2% w/v, 2% w/v and 5% w/v, 5% and 10% w/v, 10% w/v and 25% w/v, and 25% w/v and 50% w/v, or other concentrations discussed herein.

Each agent listed includes the agent and all its derivatives, salts and analogues without exclusion unless expressly otherwise indicated. For example, "succinic acid" includes succinic acid, succinate, and all their salts and analogues. The agents can be administered in different formulations for the prevention of fibrous adhesions. The formulations, methods, systems, etc., discussed herein shall be taken to include formulations comprising each agent discussed herein, whether it be used alone, or in conjunction with fucoidan (or any other fucan); or in conjunction with any other agent discussed herein; or any other agent, device, or barrier; or with any combination of drugs including fucoidan, and the agents discussed herein, and any other agent.

Unless expressly stated otherwise or clear from the context, all embodiments, aspects, features, etc., can be mixed and matched, combined and permuted in any desired manner. Unless indicated otherwise, except within the claims, the use of "or" includes "and" and vice-versa. Non-limiting terms are not to be construed as limiting unless expressly stated, or the context clearly indicates, otherwise. (For example, "including," "having," and "comprising" typically indicate "including without limitation".) Singular forms, including in the claims, such as "a," "an," and "the" include the plural reference unless expressly stated, or the context clearly indicates, otherwise.

### ANTI-SDF-1 AGENTS

The chemokines compose a large family of structurally related low-molecular-weight (6- to 14-kd) proteins that function as major regulators of leukocyte migration; activation and chemotaxis during inflammatory processes (for review see Rollins, B.J., (1997) Blood 90: 909-928). Over thirty members of this cytokine superfamily have been identified to date and broadly classified into 4 subgroups C, CC, CXC and CX3C, on the basis of the position of the NH2-terminal cysteines that form essential disulphide bonds.

Stromal-derived factor (SDF)-1 is a CXC chemokine. Two forms of SDF-1 have been identified, SDF-1a and β (together herein referred to as SDF-1), which are derived from the SDF gene by alternative splicing. The genomic sequences encoding both forms have been determined (See U.S. Patent No. 5,563,048 and U.S. Patent No. 5,756,084). SDF-1 is produced constitutively in many tissues, including bone marrow, thymus, spleen, heart, lung, muscle, kidney and liver. This contrasts with many other chemokines whose expression is highly regulated by pro-inflammatory cytokines and has led to the idea that SDF-1 plays a role in steady-state homeostatic processes, including leukocyte and hematopoietic stem cell trafficking (Nagasawa, T. et al, (1994) Proc. Natl. Acad. Sci. USA 91: 2305-2309; McGrath, K.E. et al, (1999) Dev. Biol. 213: 442-456; Tashiro, K. et al, (1993) Science 261: 600-603), B lymphopoiesis; establishment of marrow myelopoiesis during embryogenesis; neurogenesis; cardiogenesis; and blood vessel formation (Aiuti, A., et al (1999) Eur J Immunol 29: 1823-1831; Zou, Y.-R. et al (1998) Nature 393: 595-599; Tachibana, K. et al, (1998) Nature 393: 591-594).

Whereas "knockout" mice made genetically deficient for other chemokines or chemokine receptors are viable and do not show any obvious perturbations, genetic deletions of SDF-1 are lethal *in utero,* with the fetus exhibiting numerous abnormalities, including defects in the hematopoietic, cardiovascular, gastrointestinal, and neural systems as well as defects in B-cell lymphopoiesis and myelopoiesis.(Nagasawa, T. et al, (1996) Nature 382(6592): 653-658; Ma, Q. et al (1998) Proc. Natl. Acad. Sci. U.S.A. 95: 9448-9453). (Bleul C et al, (1996) Nature 382; 829; Oberlin E. et al, (1996) Nature 382: 833). SDF-1 is chemotactic for many types of mature cells involved in the inflammatory process, including T and B lymphocytes, neutrophils, monocytes, and granulocytes (Bleul C et al, (1996) Nature 382; 829; Oberlin E. et al, (1996) Nature 382: 833).

SDF-1 is structurally different from other chemokines in that it has only about 22% amino acid sequence identity with other CXC chemokines, but maintains evolutional homology with other species. SDF-1 is also different from many of the other chemokines in its apparent specificity for a single receptor, CXCR4 (previously referred to as LESTR, HUMSTER, or fusin) (Federsppiel B et al (1993) Genomics: 16: 707-712; Loetscher M et al (1994) J Biol Chem: 269: 232-237; Feng et al (1996) Science 272: 872-877), and its much broader range of action. CXCR4 is expressed on neutrophils, lymphocytes and monocytes (Bleul et al (1996) J Exp Med 184: 1101-1109; Forster R. et al (1998) J Immunol 160: 1522-1531), megakaryocytes (Wang J-F et al (1998) Blood 92: 756-764), microglial cells and astrocytes (Tanabe S et al (1997) J Immunol 159: 905-911) and dendritic cells as well as primitive hematopoietic precursor stem cells (Mohle R et al (1998) 91: 4523-4530; Aiuti et al (1999) Eur J Immunol 29: 1823-1831). CXCR4 is also expressed on cells in a wide variety of other organs and tissues, including heart, brain, spleen liver and colon (Federsppiel B et al (1993) Genomics 16: 707-712; Loetscher M et al (1994) J Biol Chem 269: 232-237; Tanabe et al (1997) J Immunol 159: 905-911; Zou Y-R et al (1998) Nature 393: 595-599; Tachibana et al (1998) Nature 393: 591-594).

The present disclosure includes methods for treating, preventing, and inhibiting fibrous adhesions such as post-surgical adhesions by delivering an anti-SDF-1 agent such as a small molecule inhibitor of SDF-1, to a site suspected of having or developing a fibrous adhesion. Representative examples of such agents include anti-SDF-1 antisense oligonucleotides (ASOs) that inhibit the translation of SDF-1 mRNA, anti-SDF-1 small molecule RNAs that inhibit the translation of SDF-1 mRNA, anti-SDF-1 siRNA's/RNAi's that inhibit the transcription of SDF-1 mRNA, anti-SDF-1 ribozymes that cleave SDF-1 mRNA, small molecule inhibitors of SDF-1 that inhibit the function of SDF-1, anti-SDF-1 binding partners such as an anti-SDF-1 aptamers and anti-SDF-1 antibodies that inhibit the function of SDF-1, and anti-SDF-1 decoy oligonucleotides.

Within certain embodiments of the disclosure, the anti-SDF-1 agents are substantially continuously exposed to the target tissue via controlled release over several hours to several days from polymeric dosage forms.

Within certain embodiments, the compound given with the anti SDF-1 agent or other anti-fibrous adhesion agent herein may be at least one of the other agents discussed herein and/or a topoisomerase inhibitor such as but not limited to camptothecin, menadione or etoposide; an anticoagulant such as but not limited to heparin or dipyridamole; an antioxidant such as but not limited to lazaroid; an antihistamine such as but not limited to ketotifen; an antiproliferative drug such as but not limited to retinoids; a fibrinolytic agent, such as but not limited to, fibrinolysin, streptokinase and urokinase; recombinant tissue plasminogen activator; a non-steroidal anti-inflammatory drug such as but not limited to ibuprofen, celecoxib; an immunosupressive drug such as but not limited to a triene macrolide antibiotic such as rapamycin; or a taxane such as but not limited to paclitaxel or docetaxel. The compound can also comprise a therapeutically effective amount of an inhibitor of another chemokine or cytokine, such as, but not limited to, a small molecular weight antagonist, or an antisense oligonucleotide, siRNA's/RNAi, neutralizing antibody directed against IL-8, MCP-1, TNF-α, IL-10 or an integrin receptor such as, but not limited to, α4β7 or α4β1. Neutralizing antibodies against SDF-1 are known and are also available commercially. The therapeutically effective amount of the SDF-1 inhibitor can be delivered as a part of a composition and the SDF-1 inhibitor can be from about 0.0001 %, 0.001, 0.01 to 1% w/w, or 0.1% to 35%, 5% to 50%, 20-80%, or 80% to 100% w/v of the composition.

The agents can further comprise placing the SDF-1 inhibitor in a biocompatible matrix, such as an hyaluronic acid film, that may adhere to the surgical area where a fibrous adhesion has potential to develop. These formulations may then release the compound(s) over a period of a few hours to few days to inhibit the inflammatory and angiogenic processes involved in fibrous adhesion formation and permit normal wound repair. Hyaluronic acid films, made flexible by the addition of 10% glycerol and crosslinked with 2mM EDAC (water soluble carboimide), are mucoadhesive, biocompatible films that may be applied to abraded surgical sites without inducing any toxicity.

The disclosure can further comprise forming a charged aqueous gel with positively charged excipients such as, for example, chitosan or poly-I-lysine, and a negatively charged SDF-1 inhibitor. Inhibitors of SDF-1 expression such as, for example, an antisense oligonucleotide, ribozyme, siRNA/RNAi, can be incorporated into such a gel for application to a disease site.

An embodiment of this invention can further comprise the use of fucans as transfection agents for nucleic acid chains able to inhibit of SDF-1 expression. The advancing area of medicine known as gene therapy is constrained by drug delivery issues whereby gene fragments or nucleic acid chains, such as oligonucleotides including ribozymes, antisense nucleotides, siRNA/RNAi's, may have their cell uptake inhibited due to the charge and large molecular weight of these compounds. The disclosure can further comprise binding or encapsulating the nucleic acid chain designed for the inhibition of SDF-1 expression, within a fucoidan microparticle. This disclosure can further comprise chemically crosslinking the particle to inhibit dissolution before application to the surgical site.

This disclosure can further comprise binding the nucleic acid chain designed for the inhibition of SDF-1 expression, with chitosan (a cationic polysaccharide), or other cationic polymer. The complex thus formed provides protection of the nucleic acid from degradation due to endogenous enzymes and results in controlled release of the nucleic acid to the site of action.

In one exemplary embodiment, the methods involve the design and synthesis of small RNAs that are complementary in sequence to a segment of mRNA and in particular the mRNA that codes for SDF-1 protein. Expression of the small RNAs can efficiently block the translation of the SDF-1 mRNA and thus eliminate the production of the chemokine. In another exemplary embodiment, the expression of SDF-1 is inhibited by the presence of specific antisense oligonucleotide sequences which can block the transcription of SDF-1 mRNA, or by administration of a specific ribozyme that can recognize and cut the mRNA encoding the chemokine.

The term "oligonucleotide" refers to an oligomer or polymer of ribonucleic acid or deoxyribonucleic acid. This term includes oligonucleotides composed of naturally occurring nucleobases, sugars and covalent intersugar (backbone) linkages as well as oligonucleotides having non-naturally-occurring portions which function similarly. Such modified or substituted oligonucleotides are often preferred over native forms because of desirable properties such as, for example, enhanced cellular uptake, enhanced binding to target or increased stability in the presence of nucleases.

Representative antisense compounds comprise from about 5 to about 50 nucleobases. Particularly common are antisense oligonucleotides comprising from about 8 to about 30 nucleobases and even more common are antisense oligonucleotides from about 15 to 25 nucleobases (*e.g.,* from about 15 to about 25 linked nucleosides). As is known, a nucleoside is a base-sugar combination. The base portion of the nucleoside is normally a heterocyclic base. The two most common classes of such heterocyclic bases are the purines and the pyrimidines. Nucleotides are nucleosides that further include at least one phosphate group covalently linked to the sugar portion of the nucleoside. For those nucleosides that include a pentofuranosyl sugar, the phosphate group can be linked to either the 2', 3' or 5' hydroxyl moiety of the sugar. In forming oligonucleotides, the phosphate groups covalently link adjacent nucleosides to one another to form a linear polymeric compound. In turn the respective ends of this linear polymeric structure can be further joined to form a circular structure, however, open linear structures are generally preferred. Within the oligonucleotides structure, the phosphate groups are commonly referred to as forming the internucleoside backbone of the oligonucleotides. The normal linkage or backbone of RNA and DNA is a 3' to 5' phosphodiester linkage.

Specific examples of preferred antisense compounds include oligonucleotides containing modified backbones or non-natural internucleoside linkages. As defined herein, oligonucleotides having modified backbones include those that retain a phosphorous atom in the backbone and those that do not have a phosphorous atom in the backbone. For the purposes of this specification, modified oligonucleotides that do not have a phosphorous atom in their internucleoside backbone can also be considered to be oligonucleotides.

Preferred modified oligonucleotide backbones include, for example, phosphorothioates, chiral phosphorothioates, phosphorodithioates, phosphotriesters, aminoalkylphosphotriesters, methyl and other alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates having normal 3'-5' linkages, 2'-5' linked analogs of these, and those having inverted polarity wherein the adjacent pairs of nucleoside units are linked 3'-5' to 5'-3' or 2'-5' to 5'-2'. Various salts, mixed salts and free acid forms are also included.

Representative United States patents that discuss the preparation of phosphorus-containing linkages include, but are not limited, to U.S. Pat. Nos. 3,687,808; 4,469,863; 4,476,301; 5,023,243; 5,177,196; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,306; 5,550,111; 5,563,253; 5,571,799; 5,587,361; and 5,625,050.

Preferred modified oligonucleotide backbones that do not include a phosphorus atom therein have backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and CH₂ component parts.

Representative United States patents that discuss oligonucleosides include, but are not limited to, U.S. Pat. Nos. 5,034,506; 5,166,315; 5,185,444; 5,214,134; 5,216,141; 5,235,033; 5,264,562; 5,264,564; 5,405,938; 5,434,257; 5,466,677; 5,470,967; 5,489,677; 5,541,307; 5,561,225; 5,596,086; 5,602,240; 5,610,289; 5,602,240; 5,608,046; 5,610,289; 5,618,704; 5,623,070; 5,663,312; 5,633,360; 5,677,437; and 5,677,439.

In some oligonucleotide mimetics both the sugar and the internucleoside linkage, i.e., the backbone, of the nucleotide units are replaced with novel groups. The base units are maintained for hybridization with an appropriate nucleic acid target compound. One such oligomeric compound, an oligonucleotide mimetic that has been shown to have excellent hybridization properties, is referred to as a peptide nucleic acid (PNA). In PNA compounds, the sugar-backbone of an oligonucleotide is replaced with an amide containing backbone, for example, an aminoethylglycine backbone. The nucleobases are retained and are bound directly or indirectly to aza nitrogen atoms of the amide portion of the backbone. Representative United States patents that discuss the preparation of PNA compounds include, but are not limited to, U.S. Pat. Nos.: 5,539,082; 5,714,331; and 5,719,262. Further discussing of PNA compounds can be found in Nielsen et al. (Science, 1991, 254, 1497-1500).

Certain embodiments of the disclosure are oligonucleotides with phosphorothioate backbones and oligonucleosides with heteroatom backbones, and for example, --CH₂--NH--O--CH₂--, --CH₂--N(CH₃)--O--CH₂- (known as a methylene (methylimino) or MMI backbone), --CH₂O----N(CH₃)--CH₂--, --CH₂-N(CH₃)--N(CH₃)--CH₂-- and --O--N(CH₃)--CH₂--CH₂- (wherein the native phosphodiester backbone is represented as --O--P--O--CH₂--) of the above-referenced U.S. Pat. No. 5,489,677, and the amide backbones of the above-referenced U.S. Pat. No. 5,602,240. Also preferred are oligonucleotides having morpholino backbone structures of the above-referenced U.S. Pat. No. 5,034,506.

Modified oligonucleotides may also contain one or more substituted sugar moieties. Preferred oligonucleotides comprise one of the following at the 2' position: OH; F; O--, S--, or N-alkyl, O-alkyl-O-alkyl, O--, S--, or N-alkenyl, or O--, S-- or N-alkynyl, wherein the alkyl, alkenyl and alkynyl may be substituted or unsubstituted C₁ to C₁₀ alkyl or C₂ to C₁₀ alkenyl and alkynyl. Particularly preferred are O[(CH₂) ₙO]ₘCH₃, O(CH₂)ₙOCH₃, O(CH₂)₂ON(CH₃)₂, O(CH₂)ₙNH₂, O(CH₂)ₙCH₃, O(CH₂)ₙ ONH₂, and O(CH₂)ₙON[(CH₂)ₙCH₃)]₂, where n and m are from 1 to about 10. Other preferred oligonucleotides comprise one of the following at the 2' position: C.₁ to C.₁₀ lower alkyl, substituted lower alkyl, alkaryl, aralkyl, O-alkaryl or O-aralkyl, SH, SCH₃, OCN, Cl, Br, CN, CF₃, OCF₃, SOCH₃, SO₂ CH₃, ONO₂, NO₂, N.₃, NH₂, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties. A preferred modification includes 2'-methoxyethoxy (2'-O--CH₂ CH₂ OCH₂, also known as 2'-O-(2-methoxyethyl) or 2'-MOE) (Martin et al., Helv. Chim. Acta 1995, 78, 486-504), *i.e.,* an alkoxyalkoxy group.

Other preferred modifications include 2'-methoxy (2'-O--CH₃), 2'-aminopropoxy (2'-OCH₂ CH₂ CH₂ NH₂) and 2'-fluoro (2'-F). Similar modifications may also be made at other positions on the oligonucleotide, particularly the 3' position of the sugar on the 3' terminal nucleotide or in 2'-5' linked oligonucleotides and the 5' position of 5' terminal nucleotide. Oligonucleotides may also have sugar mimetics such as cyclobutyl moieties in place of the pentofuranosyl sugar. Representative United States patents that discuss the preparation of such modified sugar structures include, but are not limited to, U.S. Pat. Nos. 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,658,873; 5,670,633; and 5,700,920.

Oligonucleotides may also include nucleobase (often referred to simply as "base") modifications or substitutions. As used herein, "unmodified" or "natural" nucleobases include the purine bases adenine (A) and guanine (G), and the pyrimidine bases thymine (T), cytosine (C) and uracil (U). Modified nucleobases include other synthetic and natural nucleobases such as 5-methylcytosine (5-me-C or m5c), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 8-azaguanine and 8-azaadenine, 7-deazaguanine and 7-deazaadenine and 3-deazaguanine and 3-deazaadenine. Further nucleobases include those disclosed in U.S. Pat. No. 3,687,808, those disclosed in the Concise Encyclopedia Of Polymer Science And Engineering 1990, pages 858-859, Kroschwitz, J. I., ed. John Wiley & Sons, those disclosed by Englisch et al. (Angewandte Chemie, International Edition 1991, 30, 613-722), and those disclosed by Sanghvi, Y. S., Crooke, S. T. and Lebleu, B., eds., Antisense Research and Applications 1993, CRC Press, Boca Raton, pages 289-302.

In some embodiments the nucleobases comprise 5-substituted pyrimidines, 6-azapyrimidines and N-2, N-6 and 0-6 substituted purines, including 2-aminopropyladenine, 5-propynyluracil and 5-propynylcytosine. 5-Methylcytosine substitutions have been shown to increase nucleic acid duplex stability by 0.6-1.2. °C. (Sanghvi, Y. S., Crooke, S. T. and Lebleu, B., eds., Antisense Research and Applications 1993, CRC Press, Boca Raton, pages 276-278) and are presently preferred base substitutions, even more particularly when combined with 2'-O-methoxyethyl sugar modifications.

Representative United States patents that discuss certain of the above noted modified nucleobases as well as other modified nucleobases include, but are not limited to, U.S. Pat. Nos. 3,687,808; 4,845,205; 5,130,302; 5,134,066; 5,175,273; 5,367,066; 5,432,272; 5,457,187; 5,459,255; 5,484,908; 5,502,177; 5,525,711; 5,552,540; 5,587,469; 5,594,121, 5,596,091; 5,614,617; and 5,681,941.

Another modification of the oligonucleotides involves chemically linking to the oligonucleotide one or more moieties or conjugates that enhance the activity, cellular distribution or cellular uptake of the oligonucleotide. Such moieties include but are not limited to lipid moieties such as a cholesterol moiety (Letsinger et al., Proc. Natl. Acad. Sci. USA 1989, 86, 6553-6556) such as a thiocholesterol (Oberhauser et al., Nucl. Acids Res. 1992, 20, 533-538) or an octadecylamine or hexylamino-carbonyl-oxycholesterol moiety (Crooke et al., J. Pharmacol. Exp. Ther. 1996, 277, 923-937), cholic acid (Manoharan et al., Bioorg. Med. Chem. Lett. 1994, 4, 1053-1059), a thioether, e.g., hexyl-S-tritylthiol (Manoharan et al., Ann. N.Y. Acad. Sci. 1992, 660, 306-309; Manoharan et al., Bioorg. Med. Chem. Let. 1993, 3, 2765-2770), an aliphatic chain, e.g., dodecandiol or undecyl residues (Saison-Behmoaras et al., EMBO J. 1991, 10, 1111-1118; Kabanov et al., FEBS Lett. 1990, 259, 327-330; Svinarchuk et al., Biochimie 1993, 75, 49-54), a phospholipid, e.g., di-hexadecyl-rac-glycerol or triethylammonium 1,2-di-O-hexadecyl-rac-glycero-3-H-phosphonate (Manoharan et al., Tetrahedron Lett. 1995, 36, 3651-3654; Shea et al., Nucl. Acids Res. 1990, 18, 3777-3783), a polyamine or a polyethylene glycol chain (Manoharan et al., Nucleosides & Nucleotides 1995, 14, 969-973), a adamantane acetic acid (Manoharan et al., Tetrahedron Lett. 1995, 36, 3651-3654), a palmityl moiety (Mishra et al., Biochim. Biophys. Acta 1995, 1264, 229-237).

Representative United States patents that discuss the preparation of such oligonucleotide conjugates include, but are not limited to, U.S. Pat. Nos. 4,828,979; 4,948,882; 5,218,105; 5,525,465; 5,541,313; 5,545,730; 5,552,538; 5,578,717, 5,580,731; 5,580,731; 5,591,584; 5,109,124; 5,118,802; 5,138,045; 5,414,077; 5,486,603; 5,512,439; 5,578,718; 5,608,046; 4,587,044; 4,605,735; 4,667,025; 4,762,779; 4,789,737; 4,824,941; 4,835,263; 4,876,335; 4,904,582; 4,958,013; 5,082,830; 5,112,963; 5,214,136; 5,082,830; 5,112,963; 5,214,136; 5,245,022; 5,254,469; 5,258,506; 5,262,536; 5,272,250; 5,292,873; 5,317,098; 5,371,241, 5,391,723; 5,416,203, 5,451,463; 5,510,475; 5,512,667; 5,514,785; 5,565,552; 5,567,810; 5,574,142; 5,585,481; 5,587,371; 5,595,726; 5,597,696; 5,599,923; 5,599,928 and 5,688,941.

The present disclosure also includes oligonucleotides which are chimeric oligonucleotides. "Chimeric" oligonucleotides or "chimeras," in the context of this invention, are oligonucleotides that contain two or more chemically distinct regions, each made up of at least one nucleotide. These oligonucleotides typically contain at least one region wherein the oligonucleotide is modified so as to confer upon the oligonucleotide increased resistance to nuclease degradation, increased cellular uptake, and/or increased binding affinity for the target nucleic acid. An additional region of the oligonucleotide may serve as a substrate for enzymes capable of cleaving RNA:DNA or RNA:RNA hybrids. By way of example, RNase H is a cellular endonuclease which cleaves the RNA strand of an RNA:DNA duplex. Activation of RNase H, therefore, results in cleavage of the RNA target, thereby greatly enhancing the efficiency of antisense inhibition of gene expression. Cleavage of the RNA target can be routinely detected by gel electrophoresis and, if necessary, associated nucleic acid hybridization techniques.

Examples of chimeric oligonucleotides include but are not limited to "gapmers," in which three distinct regions are present, normally with a central region flanked by two regions which are chemically equivalent to each other but distinct from the gap. An example of a gapmer is an oligonucleotide in which a central portion (the "gap") of the oligonucleotide serves as a substrate for RNase H and is composed of 2'-deoxynucleotides, while the flanking portions (the 5' and 3' "wings") are modified to have greater affinity for the target RNA molecule but are unable to support nuclease activity (e.g., fluoro- or 2'-O-methoxyethyl-substituted or locked nucleic acid). Chimeric oligonucleotides are not limited to those with modifications on the sugar, but may also include oligonucleosides or oligonucleotides with modified backbones, e.g., with regions of phosphorothioate (P=S) and phosphodiester (P=O) backbone linkages or with regions of MMI and P=S backbone linkages.

Other chimeras include "wingmers," also known as "hemimers," that is, oligonucleotides with two distinct regions. In a preferred example of a wingmer, the 5' portion of the oligonucleotide serves as a substrate for RNase H and is preferably composed of 2'-deoxynucleotides, whereas the 3' portion is modified in such a fashion so as to have greater affinity for the target RNA molecule but is unable to support nuclease activity (e.g., 2'-fluoro- or 2'-O-methoxyethyl-substituted), or vice-versa.

According to the disclosure, one, a plurality, or all of the nucleotide subunits of the oligonucleotides of the invention may bear a 2'-O-methoxyethyl (--O--CH₂ CH₂ OCH₃) modification. Oligonucleotides comprising a plurality of nucleotide subunits having a 2'-O-methoxyethyl modification can have such a modification on any of the nucleotide subunits within the oligonucleotide, and may be chimeric oligonucleotides. Aside from or in addition to 2'-O-methoxyethyl modifications, oligonucleotides containing other modifications which enhance antisense efficacy, potency or target affinity are also preferred. Chimeric oligonucleotides comprising one or more such modifications are presently preferred.

The oligonucleotides used in accordance with this disclosure may be conveniently and routinely made through the well-known technique of solid phase synthesis. Equipment for such synthesis is sold by several vendors including Applied Biosystems. Any other desired approach for such synthesis may also be employed. For example, it is well known to use similar techniques to prepare oligonucleotides such as the phosphorothioates and 2'-alkoxy or 2'-alkoxyalkoxy derivatives, including 2'-O-methoxyethyl oligonucleotides (Martin, P., Helv. Chim. Acta 1995, 78, 486-504). It is also well known to use similar techniques and commercially available modified amidites and controlled-pore glass (CPG) products such as biotin, fluorescein, acridine or psoralen-modified amidites and/or CPG (available from Glen Research, Sterling, Va.) to synthesize fluorescently labeled, biotinylated or other conjugated oligonucleotides.

The antisense compounds of the present disclosure include bioequivalent compounds, including pharmaceutically acceptable salts and prodrugs. This is intended to encompass any pharmaceutically acceptable salts, esters, or salts of such esters, or any other compound which, upon administration to an animal including a human, is capable of providing (directly or indirectly) the biologically active metabolite or residue thereof. Accordingly, for example, the disclosure is also drawn to pharmaceutically acceptable salts of the nucleic acids of the invention and prodrugs of such nucleic acids. "Pharmaceutically acceptable salts" are physiologically and pharmaceutically acceptable salts of the nucleic acids of the invention: i.e., salts that retain the desired biological activity of the parent compound and do not impart undesired toxicological effects thereto (see, for example, Berge et al., "Pharmaceutical Salts," J. of Pharma Sci. 1977, 66, 1-19).

For oligonucleotides, examples of pharmaceutically acceptable salts include but are not limited to (a) salts formed with cations such as sodium, potassium, ammonium, magnesium, calcium, polyamines such as spermine and spermidine, etc.; (b) acid addition salts formed with inorganic acids, for example hydrochloric acid, hydrobromic acid, sulfuric acid, phosphoric acid, nitric acid and the like; (c) salts formed with organic acids such as, for example, acetic acid, oxalic acid, tartaric acid, succinic acid, maleic acid, fumaric acid, gluconic acid, citric acid, malic acid, ascorbic acid, benzoic acid, tannic acid, palmitic acid, alginic acid, polyglutamic acid, naphthalenesulfonic acid, methanesulfonic acid, p-toluenesulfonic acid, naphthalenedisulfonic acid, polygalacturonic acid, and the like; and (d) salts formed from elemental anions such as chlorine, bromine, and iodine.

The oligonucleotides of the disclosure may additionally or alternatively be prepared to be delivered in a "prodrug" form. The term "prodrug" indicates a therapeutic agent that is prepared in an inactive form that is converted to an active form (i.e., drug) within the body or cells thereof by the action of endogenous enzymes or other chemicals and/or conditions. For example, prodrug versions of the oligonucleotides of the invention are prepared as SATE [(S-acetyl-2-thioethyl) phosphate] derivatives according to the methods disclosed in WO 93/24510.

Certain antisense oligonucleotide sequences for the inhibition of SDF-1 protein are given in Example 3, below.

Antisense oligonucleotides having a greater or lesser number of substituent nucleotides, or that extend further along the SDF-1 mRNA in either the 3' or the 5' direction than the embodiments given in Example 3 and identified with Sequence ID Numbers 1 through 13 inclusively, but which also inhibit SDF-1 protein expression are also within the scope of this invention.

In another exemplary embodiment, the action of SDF-1 can be inhibited by the presence of specific neutralizing antibodies.

In still another embodiment the present invention provides for the use of fucans as transfection agents for nucleic acid chains designed for the inhibition of SDF-1 expression. The advancing area of medicine known as gene therapy is constrained by drug delivery issues whereby gene fragments or nucleic acid chains, such as oligonucleotides including ribozymes, antisense nucleotides and RNA inhibitors, may have their cell uptake inhibited due to the charge and large molecular weight of these compounds. Recently, the use of microparticles (such as calcium phosphate) containing the gene or nucleic acids have been proposed as transfection agents so that they bind to the cell surface and are taken up by endocytosis or invagination, resulting in cellular entry of the gene or nucleic acid. Most cells contain fucan receptors on the membrane surface. In this embodiment, the nucleic acid chain designed for the inhibition of SDF-1 expression can be bound or encapsulated within a fucoidan microparticle and the particle can be chemically crosslinked to inhibit dissolution before application to the surgical site.

Unless expressly indicated otherwise, the use of "or" includes "and" and vice-versa. Non-limiting terms are not to be construed as limiting unless expressly stated, or the context clearly indicates, otherwise. (For example, "including," "having," and "comprising" typically indicate "including without limitation".) Singular forms, such as "a," "an," and "the" include the plural reference unless expressly stated, or the context clearly indicates, otherwise.

### DISCUSSION OF QUANTITATIVE EFFECTIVENESS OF ANTI-FIBROUS ADHESION AGENTS:

In one embodiment, the efficacy of the given drug or drug combination can be assessed as a reduction of the average Total Adhesion Value (strength x area; "TAV") of the drug or combination versus a given standard, for example a drug-loaded sodium hyaluronate film versus a sham or a sodium hyaluronate film alone in the rat cecal-sidewall model for surgical fibrous adhesions. Other standards can include other films, solutions, etc., and other models, such as rabbit uterine horn model or effectiveness in humans. In various embodiments, the drugs can have an average TAV less than or equal to 0.01%, 1%, 5%, 10%, 25%, 50%, or 75% of the control's value, for example the hyaluronate film alone, using the rat cecal-sidewall model for surgical fibrous adhesions. In other measurement parameters, the drugs can inhibit substantially all fibrous adhesion formation in a patient.

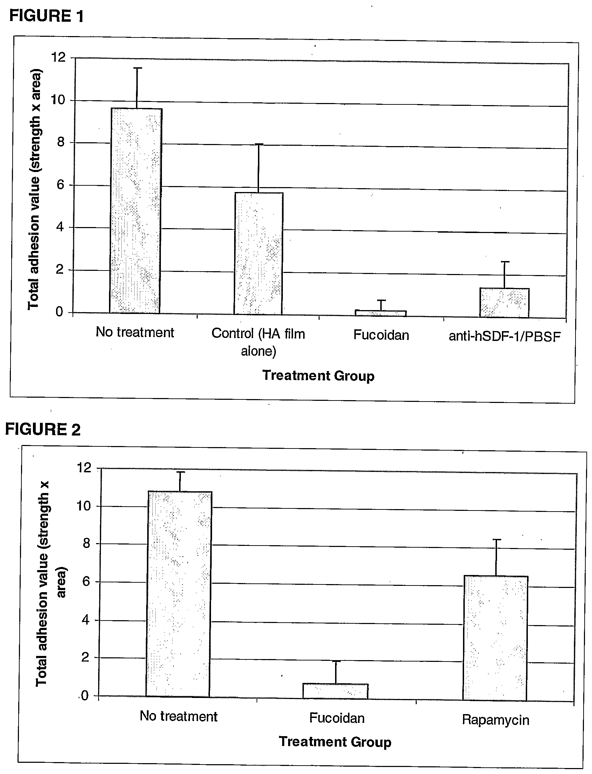

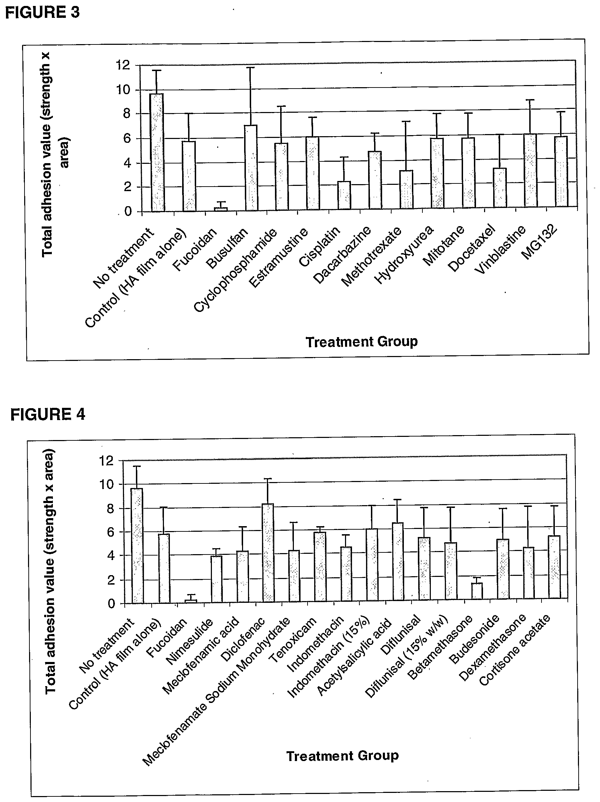

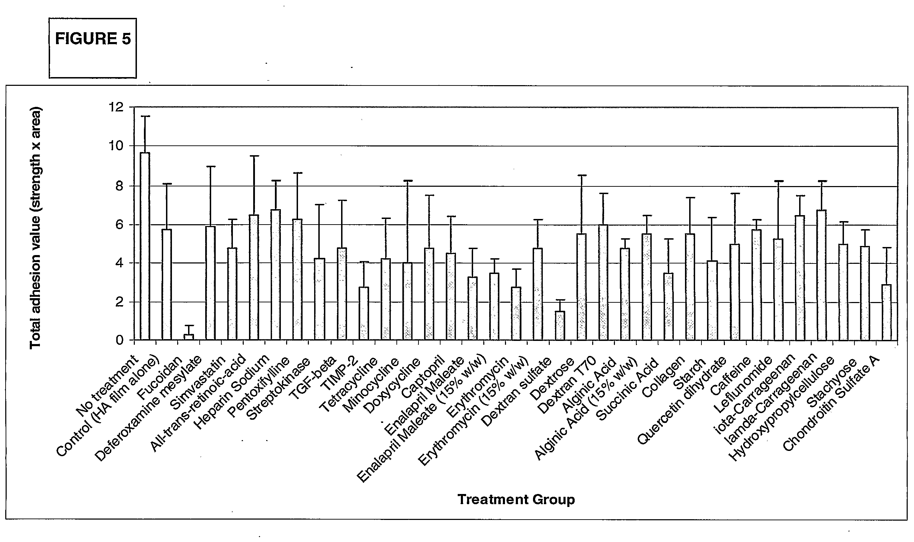

For illustration, in the Examples below comparing drug efficacy against a sodium hyaluronate film alone in the rat cecal-sidewall model for surgical fibrous adhesions, and as shown in Figures 1-5, fucoidan had a TAV of less than about 10% (even down to about 0%), anti-hSDF-1/PBSF antibody and betamethasone had a TAV of less than about 25%, chondroitin sulfate A, dextran sulfate, erythromycin, and TIMP-2 had a TAV of less than about 50%, streptokinase, tetracycline, minocycline, enalapril maleate, succinic acid, starch, methotrexate, docetaxel, nimesulide, meclofenamic acid, meclofenamate sodium monohydrate, and dexamethasone had a TAV of less than about 75%, budesonide, diflunisal, dacarbazine, stachyose, hydroxypropylcellulose, indomethacin, quercetin, alginic acid, captopril, doxycycline, TGF-beta, and simvastatin had a TAV of less than about 90%, and cortisone acetate, tenoxicam, cyclophosphamide, leflunomide, collagen, dextrose had a TAV of less than about 100%. In another embodiment, the drug(s) can be assessed according their efficacy in inhibiting all adhesions (*i.e.,* scored a zero on the Total Adhesion Value (strength x area) scale) in at least one test subject or patient. For illustration, in the examples below, each of fucoidan, cisplatin, methotrexate, docetaxel, dexamethasone, and anti-SDF-1 antibody completely inhibited fibrous adhesions, in at least one test animal after administering a therapeutically effective amount of the agent to the cecal-sidewall site suspected of developing fibrous adhesions.

The present invention includes the use of the sulfated polysaccharides known as fucans (including derivatives and analogues thereof and regardless of the source) for the treatment or prevention of fibrous adhesions, rheumatoid arthritis, and psoriasis (where treatment as used herein includes both the treatment of existing conditions and the inhibition of potential conditions). As demonstrated in the Examples below, fucans inhibit cell proliferation, inflammatory responses/events and angiogenesis, including for example in surgical adhesions and other fibrous adhesions.

In certain embodiments the present invention includes treatment, inhibition, etc., using fucans such as fucoidan, with low sulphate densities, which may reduce anticoagulant effects. In particular, it was found that fucoidan that had an average of about 1 or less sulphate group per fucose monomer (and typically less than a ratio of about 1.4) was an effective agent for the prevention of surgical adhesions and that the therapeutic window was high. Accordingly, this invention comprises fucans with sulphate to fucose ratios of less than about 1.0, 0.9 or less for treating inflammatory disease including psoriasis and arthritis, and including fibrous adhesions, including surgical adhesions.

### EXAMPLES

To briefly summarize, Examples 1 and 2 are directed to an analysis of the efficacy of various agents against surgical adhesions using animal models. Examples 3-7 relate to antisense and other SDF-1 inhibitors. Examples 8-10 relate to rapamycin. Examples 11-14 relate to various formulations for anti-fibrous adhesion agents. Example 15 relates to the efficacy of fucoidan from different sources.

### Example 1: Efficacy of Drug Loaded Hyaluronic Acid Film for the Prevention of Surgical Adhesions Using of the Caecal-Sidewall Surgical Adhesion Model in Rats.

The rat caecal sidewall model of surgical adhesions was used to investigate the effect of administration of each agent discussed herein (hereafter referred to as the drug) for the prevention of post-surgical type of fibrous adhesions. In this model, rats were divided into groups of 4. After surgical trauma, the rats were either untreated, treated with crosslinked hyaluronic acid (HA) film, or crosslinked HA film containing drug at the following concentrations in the film (% w/w):

### MISCELLANEOUS

**SDF-1 Inhibitors**

| | | |
|---|---|---|
| Anti-hSDF-1/PBSF antibody | R&D Scientific | 250 ppm |

**Triene Macrolide Antibiotic**

| | | |
|---|---|---|
| Rapamycin | AG Scientific | 1.6% w/w |

**Iron-Chelating Agent**

| | | |
|---|---|---|
| Deferoxamine Mesylate | Sigma | 5% w/w |

**3-Hydrox-3-Methylgluteryl-CoA Reductase Inhibitors**

| | | |
|---|---|---|
| Simvastatin | Aldrich | 5% w/w |

**Retinoids**

| | | |
|---|---|---|
| all-trans-Retinoic Acid | Aldrich | 5% w/w |

**Antithrombotics**

| | | |
|---|---|---|
| Heparin Sodium | Hepalean® Organon | 4 USP units/mg |

**Anticoagulants**

| | | |
|---|---|---|
| Pentoxifylline | Sigma | 5% w/w |

**Plasminogen Activators**

| | | |
|---|---|---|
| Streptokinase | Sigma | 25 units/mg |

**Cytokines**

| | | |
|---|---|---|
| TGF-β | R&D Systems | 2.5 ppm |

**Matrix Metalloproteinase Inhibitor**

| | | |
|---|---|---|
| TIMP-2 | Sigma | 12.5 ppm |

**Tetracyclines**

| | | |
|---|---|---|
| Tetracycline HCl | Sigma | 15% |
| Minocycline Hydrochloride Salt | Sigma | 5% w/w |
| Doxycycline HCl | Sigma | 5% w/w |

**ACE inhibitors**

| | | |
|---|---|---|
| Captopril | Sigma | 5% w/w |
| Enalapril Maleate | Sigma | 5% w/w |
| Enalapril Maleate | Sigma | 15% w/w |

**Dextran Sugars**

| | | |
|---|---|---|
| Dextran Sulfate | Sigma | 5% w/w |
| Dextrose | Merck | 5% w/w |
| Dextran T70 | Amersham | 5% w/w |

**Miscellaneous**

| | | |
|---|---|---|
| Erythromycin | Sigma | 5% w/w |
| Erythromycin | Sigma | 15% w/w |
| Alginic Acid | Sigma | 5% w/w |
| Alginic Acid | Sigma | 15% w/w |
| Succinic Acid | Sigma | 5% w/w |
| Collagen | Sigma | 5% w/w |
| Starch | BDH Chemicals | 5% w/w |
| Quercetin Dihydrate | Sigma | 5% w/w |
| Caffeine | BDH Chemicals | 5% w/w |
| Leflunomide | Sigma | 5 % w/w |
| Hydroxypropylcellulose | Aldrich | 5% w/w |
| Stachyose | Sigma | 5% w/w |
| Chondroitin Sulfate A | Calbiochem | 5% w/w |

**Carrageenans**

| | | |
|---|---|---|
| ?-Carrageenan | Fluka | 5% w/w |
| ?-Carrageenan | Sigma | 5% w/w |

### ANTINEOPLASIC AGENTS

**Alkylating Agents**

| | | |
|---|---|---|
| Busulfan | Sigma | 5% w/w |
| Cyclophosphamide | Aldrich | 2% w/w |
| Estramustine | Kabi Pharmacia | 5% w/w |
| Cisplatin | Faulding | 2% w/w |
| Dacarbazine | Sigma | 5% w/w |

**Antimetabolite**

| | | |
|---|---|---|
| Methotrexate | Sigma | 2% w/w |

**Ribonucleotide Reductase Inhibitor**

| | | |
|---|---|---|
| Hydroxyurea | Aldrich | 5% w/w |

**Cytotoxic Antibiotic**

| | | |
|---|---|---|
| Mitotane | Aldrich | 5% w/w |

**Taxanes**

| | | |
|---|---|---|
| Docetaxel | Aldrich | 5% w/w |

**Vinca Alkyloid**

| | | |
|---|---|---|
| Vinblastine Sulfate | Biochemika | 5% w/w |

**Protease Inhibitor**

| | | |
|---|---|---|
| MG132 | Sigma | 1.25% w/w |

### ANTI-INFLAMMATORY AGENTS

**COX-2 Inhibitors**

| | | |
|---|---|---|
| Nimesulide | Sigma | 5% w/w or 15% |

**Fenamates**

| | | |
|---|---|---|
| Meclofenamic Acid | Sigma | 15% w/w |
| Diclofenac | Novartis | 0.7% w/w |
| Meclofenamate | Warner Lambert | 5% w/w |
| Sodium | Company | |
| Monohydrate | | |

**Oxicams**

| | | |
|---|---|---|
| Tenoxicam | Sigma | 5% w/w |

**Acetyl Acid Derivatives**

| | | |
|---|---|---|
| Indomethacin | Sigma | 5% w/w |
| Indomethacin | Sigma | 15% w/w |
| *Salicylic Acid Derivatives* | | |
| Acetylsalicylic Acid | Sigma | 5% w/w |
| Diflusinal | Sigma | 5% w/w |
| Diflusinal | Sigma | 15% w/w |

**Corticosteroids**

| | | |
|---|---|---|
| Betamethasone | Sigma | 15% w/w |
| Budesonide | Sigma | 5% w/w |
| Dexamethasone | Sigma | 5% w/w |
| Cortisone Acetate | Sigma | 5% w/w |

Preparation of Hyaluronic Acid Films. Solutions of hyaluronic acid were prepared by dissolving sodium hyaluronate and glycerol in water overnight. The ratio of sodium hyaluronate to glycerol was about 3:1, and the total concentration of solute (sodium hyaluronate and glycerol) was between 2 and 3% w/w. The drug was incorporated into the solution by mixing with a spatula in sufficient amount to produce a 2%, 5%, 15,% or about 30% w/w mixture of the drug relative to the sodium hyaluronate and glycerol (*i.e.,* the drug concentration does not include the water in the calculations.

The crosslinking agent EDAC was included at about 0.1% w/w (final concentration in water). Films were cast from these solutions by pipetting the solution into 2. plastic Petri dishes and drying for at least 12 hours at 60° C. Each dried film was then carefully removed from the Petri dish using a surgical blade and cut into rectangles of 1.2 cm x 1.8 cm size.

Animal Studies. Surgical trauma was induced as follows: mature Sprague Dawley rats, each weighing 225-300 g were obtained from the University of British Columbia Animal Facility. Only animals that appeared grossly normal (i.e., showing a clean unruffled coat, bright clear eyes and an active posture) were used in the study. Animals were randomly assigned to treatment groups, weighed and anesthetized with isoflurane gas. The abdomen was shaved and cleaned with a skin-antibacterial cleanser (Steri-Stat 2%) and wiped with a chlorohexane soaked gauze. A nick was made in one of the tail veins to elicit a small amount of blood (< 100 mL). A white blood cell count was performed on this blood sample. An antibiotic (40,000 IU/kg of depo-penicillin) was injected into the right thigh and an analgesic (0.01 mg/kg of buprenorphine) was injected into the left thigh of each rat. A 4 cm incision was made in the skin beginning approximately 2 cm caudal to the linea alba while the muscle was tended with forceps. The caecum was located, pulled out of the abdominal cavity and scraped 45 times on both the ventral and dorsal surfaces with a number 10 scalpel held at a 45 degree angle relative to the caecum surface. Scraping was in the opposite direction to which the blade was pointing. The scraped caecum was wrapped in saline-soaked gauze. Doynes were used to separate the peritoneal wall from the skin, and the peritoneal wall was inverted using the doynes to expose the inside of the wall. A rectangular injury roughly 1.2 cm by 1.8 cm was made by shallow incisions to the peritoneal wall. The top membrane and a layer of muscle tissue was removed using forceps. The caecum was stitched to the four corners of the rectangle using a 5-0 suture, and without tying off the top two stitches. A piece of film was placed onto the abraded rectangle and then the top two stitches were tied firmly. In the case of the untreated control group, no film was placed over the abraded site. The exposed organs were replaced in the abdomen in such a way as to prevent torsional stress on the intestines. The abdominal wall was closed with 5-0 sutures and the surgical incision was closed with 3-0 sutures. A collar was placed around the neck of the animal to prevent it from interfering with the stitches. The rat was placed in a clean cage and warmed with a heating lamp until consciousness was regained. The rats were weighed daily following the surgery.

One week after surgery the fibrous adhesions were assessed. The incision site was visually checked for signs of inflammation or lack of wound healing. The rats were anaesthetized and blood samples were taken for white blood cell determination from a tail vein nick. Then the rats were sacrificed using CO₂ and then re-opened along the midline. The internal organs were visually checked for anomalies. The sutures were cut and fibrous adhesions between the caecum and the sidewall as well at the stitching points were assessed and scored according to a predefined scoring system. There were two criterion used in this assessment. The fibrous adhesions were determined according to the following scales:
Area Covered by Adhesions Scale:

| | |
|---|---|
| 1-25% | 1 |
| 25-50% | 2 |
| 51-75% | 3 |
| 76-100% | 4 |

Strength of Adhesion Scale:

| | |
|---|---|
| 0 | no adhesions |
| 1 | adhesions separable by blunt dissection |
| 2 | adhesions not easily separable |
| 3 | sharp dissection of adhesion required (tearing of the wall or horn) |

The overall fibrous adhesion score for an animal was determined by multiplying the area score by the strength of fibrous adhesion score.

A set of graphs showing the effect of treatment by drug loaded film is given as Figures 1-5. The data demonstrate that animals treated with the following drugs at the loading levels given were found to have a lower overall fibrous adhesion score than those animals in the control groups, demonstrating the effective inhibition of fibrous adhesion formation by drug loaded films. The reduction in adhesion score was statistically significant (p<0.05) using a one-tailed Student's t-test for dextran sulfate (5% w/w), enalapril maleate (5% w/w), cisplatin (2% w/w), dextran sulfate (25% w/w), fucoidan (33% w/w), erythromycin (5% w/w), and tetracycline (5% w/w).

### Example 2: Efficacy of Drug Loaded Hyaluronic Acid Film for the Prevention of Surgical Adhesions Using of the Uterine Horn Surgical Adhesion Model in Rabbits.

The rabbit uterine horn model of surgical adhesions is used to investigate the effect of administration of an agent selected discussed herein (hereafter referred to as the drug) for the prevention of post-surgical type of fibrous adhesions. In this model, rabbits are divided into groups of 4. After surgical trauma, the rabbits are either treated with crosslinked hyaluronic acid (HA) film, crosslinked HA film containing the drug at 5% w/w loading, crosslinked HA film containing the drug at 2% w/w loading, crosslinked HA film containing the drug at about 30% w/w loading, crosslinked HA film containing drug at any concentration between 0.5% w/w and 99% w/w loading, or are untreated (control group). Other treatment groups include solutions (or suspensions) of the drug at concentrations of between 0.0001% w/v and 1% w/v, or solutions (or suspensions) of the drug at concentrations between 1% w/v and 2% w/v, or solutions (or suspensions) of the drug at concentrations of between 2% w/v and 5% w/v, or solutions (or suspensions) of the drug at concentrations between 5% and 10% w/v, or solutions (or suspensions) of the drug at concentrations between 10% w/v and 25% w/v, or solutions (or suspensions) of the drug at concentrations between 25% w/v and 50% w/v.

Preparation of Hyaluronic Acid Films. Solutions of hyaluronic acid are prepared by dissolving sodium hyaluronate and glycerol in water overnight. The ratio of sodium hyaluronate to glycerol is about 3:1, and the total concentration of solute (sodium hyaluronate and glycerol) is between 1 and 2.5% w/w. The drug is incorporated into the solution by mixing with a spatula in sufficient amount to produce a 2%, 5%, or about 30% w/w mixture of the drug relative to the sodium hyaluronate and glycerol (*i.e.,* the drug concentration does not include the water in the calculations.

The crosslinking agent EDAC will be included at about 0.1% w/w (final concentration). Films are cast from these solutions by pipetting the solution into 2. plastic Petri dishes and drying for at least 12 hours at 60° C. Each dried film is then carefully removed from the Petri dish using a surgical blade and cut into rectangles of 1.2 cm x 1.8 cm size

Preparation of Drug Solutions (Instillates) Appropriate amounts of the drug are dissolved in aqueous solutions (e.g. Lactated Ringers Solution USP). These solutions are filtered to remove coarse particulate matter and are sterilized by filtration through a 22 µm filter, or by autoclave, or other suitable means. If the drug is administered as a suspension rather than a solution then no filtering step is used. The sterilized instillate drug solutions are administered directly to the abdominal cavity at the end of the surgical procedure, just prior to the final suturing of the rabbit to close the surgical incision.

### Animal Studies

These formulations are tested using a uterine horn surgical adhesion model in rabbits. Briefly, an incision is made in the abdomen of the rabbits. The uterine horns are located and injured by clamping near the base of the horn for a prescribed (and consistent) length of time. The peritoneal sidewall of the rabbit is injured in a specified area by abrasion with a scalpel. The uterine horns are then placed in such a way that they lay on the abraded area of the peritoneal sidewall and are stitched at the tip of the horn. The stitch is outside the abraded area of the sidewall but prevents the uterine horn from contracting away from the abraded sidewall area.

The efficacy of the film formulations are evaluated by placing the film directly on the abraded sidewall area (between the horn and the sidewall).

The efficacy of the instillate formulations are evaluated by instilling 30 mL of the formulation being tested into the abdomen of the rabbit prior to completing the surgical procedure.

These formulations are compared with a control group which is treated by the instillation of 30 mL of Lactated Ringer's Injection USP into the abdomen of the rabbits prior to completing the surgical procedure.

At 14 days after the procedure the rabbits are euthanized and the extent of adhesion formation is evaluated. The evaluator is blinded as to which group is being evaluated. These adhesions are rated as a product of the area covered by the adhesions and the strength of the adhesions that form. The area covered by adhesions is rated on a 4 point scale and the strength of the adhesions is rated on a scale of 0 to 3. The following scales are used:
Strength of Adhesion Rating Scale:

| | |
|---|---|
| 0 | no adhesions |
| 1 | adhesions separable by blunt dissection |
| 2 | adhesions not easily separable |
| 3 | sharp dissection of adhesion required (tearing of the wall or horn) |

Area Covered by Adhesions Scale:

| | |
|---|---|
| 1-25% | 1 |
| 25-50% | 2 |
| 51-75% | 3 |
| 76-100% | 4 |

An adhesion score for each rabbit is then obtained by multiplying the scores for the strength of the adhesions by the scores for the area covered by the adhesion. Animals treated with the drug are found to have a significantly lower overall fibrous adhesion score than those animals in the control groups, demonstrating the effective inhibition of fibrous adhesion formation by drug loaded films and/or drug loaded instillate solutions (or suspensions) with no obvious toxicity as observed by the lack of alteration in the appearance, weight, or white blood cell count in the treated rats when compared to the control group.

### EXAMPLE 3: DETERMINATION OF SUITABLE ANTISENSE OLIGONUCLEOTIDES FOR THE INHIBITION OF SDF-1 PROTEIN

In order to determine potential antisense sequences for SDF-1 mRNA, the mRNA sequence for SDF-1 was first obtained from the National Center for Biotechnology Information (NCBI) database. The database can be accessed at http://www.ncbi.nlm.nih.gov. In the search parameter, "SDF" was entered and a sequence with accession number NM_000609 corresponding to human SDF-1 mRNA was found

The sequence was submitted to mfold, an online service which predicts RNA or DNA secondary sequences and can be accessed at http://bioweb.pasteur.fr/seqanal/interfaces/mfold-simple.html. Note that the mfold service is limited to 3000 bases while the SDF-1 sequence is 3541 bases long. Only the first 2760 bases of the SDF-1 sequence were used.

The mfold service takes approximately 48 hours to process a sequence and posts the results online for retrieval. The results should be downloaded as they are deleted from the mfold server within 7 days.

The mfold service predicted 38 potential structures and each structure was looked at for potential sites to which an antisense sequence could bind to the mRNA. This was done by looking for loops in the structure, as these regions had no intramolecular binding, as depicted below:
ATTGT 5'-CAG A 3'-GTC G GGCCC [SEQ ID. NO. 1]

The complementary sequence that would be used for the above loop was 5'-CAGCCGGGCTACAATCTG [SEQ ID. NO. 2]. In all, 12 sequences were designed based on this method. It was also confirmed that each loop structure used to design an antisense was present in at least 19 of the 38 sequences.

**Table 1: Exemplary oligonucleotide sequences for the inhibition of SDF-1 Protein production**

| Antisense Oligonucleotide Sequence | Seq ID No. |
|---|---|
| 5'-CAG CCG GGC TAC AAT CTG-3' | 2 |
| 5'-GCC AGT GAC ACT GAA TAA-3' | 3 |
| 5'-GCT GCT ACG TGT CGC CAG T-3' | 4 |
| 5'-ACG TGT CGC CAG TGA CAC TGA-3' | 5 |
| 5'-GGC TGG GTC TCA CTC TGC C-3' | 6 |
| 5'-GAA CGT GGA GGA TGT GGA GG-3' | 7 |
| 5'-CAG GAT TGG TTA TTT TGT-3' | 8 |
| 5'-AGA TGT GAA TTG GGA AAG AA-3' | 9 |
| 5'-AAG ATG AGG TTA GAT GTG AA-3' | 10 |
| 5'-GAG GTT AGA TGT GAA TTG GGA-3' | 11 |
| 5'-AAT AAT TTT CCC CTG CAG TTT-3' | 12 |
| 5'-AGG AAT TGT TAT CCA AAT AAT-3' | 13 |

The 12 sequences were then synthesized by NAPS at the University of British Columbia for further testing in an appropriate cell culture model.

### EXAMPLE 4: MANUFACTURE OF AND CONTROLLED RELEASE OF SDF-1 ANTISENSE OLIGONUCLEOTIDE FROM POLYCAPROLACTONE PASTE

The SDF-1 inhibitor is blended into polycaprolactone (PCL, Birmingham polymers, molecular weight 54K) at 60°C by spatula levigation at a concentration of 10% (w/w). This mixture is then loaded into 1 ml plastic syringes and allowed to cool. This formulation can be injected through an 18 gauge needle at 56°C.

To measure drug release from the PCL paste, 10 mg aliquots of molten paste are injected onto the base of 15 mL glass tubes and allowed to cool and set. Fifteen mL of phosphate buffered saline (PBS) are added to the tubes and the tubes are capped, and tumbled end over end in a 37°C oven. At specified times, the tubes are removed and the amount of drug released is analyzed by absorbance spectroscopy. The release of the SDF-1 inhibitor is characterized by an initial burst of drug release followed by a slow sustained release. This dosage form of the SDF-1 inhibitor represents a biocompatible, biodegradable, injectable formulation of inhibitor that releases the drug in a controlled manner.

### EXAMPLE 5: THE EFFECT OF SDF-1 INHIBITOR (SPECIFIC ANTISENSE OLIGONUCLEOTIDE (ASO), RIBOZYME, mRNA INHIBITOR OR NEUTRALIZING ANTIBODY)-LOADED PELLETS ON ANGIOGENESIS IN THE CHORIOALLANTOIC MEMBRANE OF THE CHICK EMBRYO (CAM ASSAY).

Fertilized chicken eggs are obtained from a local hatchery and placed in an incubator with an automatic rotator at 37° C for 3 and ½ days. The eggs are manually rotated in the incubator such that their sharp end is facing up for 5-10 minutes to allow detachment of the egg contents from the inner membrane. Using 70% ethanol and Kimwipes, the entire eggshell is wiped down to help clean and sanitize the outside of the egg. Inside a laminar flow hood, the egg is held with the blunt side up and a hole is made in the blunt end of the egg by carefully cracking the shell with the end of forceps. The shell remnants are gently removed with forceps to form a hole in the blunt end. This circular hole can be made as large as 2 to 3 cm in diameter without damaging the inner membrane. Once the hole is created in the shell, the inner shell membrane (which houses the egg contents) is gently torn and removed using the forceps, taking care not to damage the chorioallantoic membrane (CAM) (which houses the yolk and developing chick embryo).

The hole is then covered with the sheet of sterilized parafilm wax paper by gently stretching the parafilm and placing it around the hole. The egg is then placed in the egg rack in the incubator (37° C) and positioned in such a way as to prevent rotation. After 6 days each egg is removed one by one from the incubator (blunt side up), and the parafilm covering the window is removed for direct access to the CAM, which originates from the hindgut of the embryo. Polymeric pellets containing the SDF-1 inhibitor (loaded between 1% - 30% w/w) are placed onto the growing capillary bed of the CAM. The egg contents are then resealed with a parafilm sheet and the egg is placed back into the 37° C incubator. After 2 more days, analysis of the CAM vasculature is recorded (48 hours after placing the drug onto the CAM capillary bed). The effect of the drug on the CAM is rated using an avascular scale, which grades the effect of the drug as 0, 1, 2, or 3. The values of the avascular scale describe the following:

| | |
|---|---|
| 0 | No antiangiogenic activity |
| 1 | Microvessel reduction |
| 2 | Small avascular zone measuring the size of the drug pellet (2mm in diameter) |
| 3 | Avascular zone measuring 4-5mm in diameter. |

The presence of the SDF-1 inhibitor-loaded pellet prevents or decreases angiogenesis in the CAM assay, which demonstrates an antiangiogenic activity of the SDF-1 inhibitor and show that a polymeric slow release formulation of this inhibitor is an effective method of releasing therapeutically effective concentrations of the drug without inducing undue toxicity.

### EXAMPLE 6: EFFICACY OF A SDF-1 INHIBITOR (SPECIFIC ASO, RIBOZYME, OR MRNA INHIBITOR) ON SDF-1 EXPRESSION IN ACTIVATED ENDOTHELIAL CELLS

Human umbilical cord endothelial cells (HUVECs) are isolated, pooled and established in primary cultures in M199 medium (Sigma-Aldrich) containing 10% FCS, 8% pooled human serum, 50 mg/mL endothelial cell growth factor, 10 U/mL heparin and antibiotics and serially passaged. One day before addition of cytokine, the cells are preseeded on fibronectin coated plates and then stimulated for 18 hours with culture media supplemented with TNF-? (2 ng/mL, R&D Systems) in the presence or absence of the SDF-1 inhibitor.

Following cytokine stimulation in the presence of SDF-1 inhibitor, the cells are harvested, lysed, and the mRNA extracted using a commercially available mRNA purification kit (Qiagen) and a reverse transcription procedure performed, also using a commercially available kit. The resulting DNA is amplified by use of the polymerase chain reaction (PCR), and quantitated on a real time PCR machine (Lightcycler, BioRad). In the case of those cultures in which a specific ribozyme or inhibitory mRNA sequence is added to inhibit SDF-1 expression, the production of SDF-1 is quantitated by the use of a commercially available SDF-1 ELISA kit (R&D Systems). Administration of the SDF-1 inhibitor prevents the expression of this chemokine in TNF-? stimulated cultures.

### EXAMPLE 7: ENCAPSULATION OF AN SDF-1 INHIBITOR (SPECIFIC ANTISENSE OLIGONUCLEOTIDE, RIBOZYME, MRNA INHIBITOR OR NEUTRALIZING ANTIBODY) IN CHITOSAN FILMS

The SDF-1 inhibitor is dissolved in 1.2 mL of dimethyl sulphoxide and then pipetted into 4 mL of a 2.5% w/v chitosan (Fluka scientific, low molecular weight) solution in 2% w/v acetic acid. This mixture is stirred by spatula for five minutes to homogeneously suspend the precipitated drug in the chitosan solution. Four mL of this viscous mixture is then poured into 2.5 cm plastic petri dishes and dried at 37° C overnight. The chitosan dries to thin films that are removed from the petri dishes. These films are moderately flexible, about 35 mm thick and with the inhibitor suspended uniformly in the chitosan matrix at a concentration of 10% (w/w relative to chitosan). To measure drug release from these chitosan films, 20 mg pieces are placed into 10 mL of PBS pH 7.4 in capped tubes and tumbled for specific times at 37° C. The amount of the inhibitor released from the films into the PBS is measured by absorbance at 260 nm. The release of the drug is characterized by an initial burst followed by a slow sustained release. This dosage form of the inhibitor represents a biocompatible, mucoadhesive formulation of SDF-1 inhibitor that releases the inhibitor in a controlled manner.

### EXAMPLE 8: MANUFACTURE OF AND CONTROLLED RELEASE OF RAPAMYCIN FROM POLYCAPROLACTONE PASTE

The rapamycin is blended into polycaprolactone (PCL, Birmingham polymers, molecular weight 54K) at 60°C by levigation with a spatula at a concentration of 10% (w/w). This mixture is then loaded into 1 ml plastic syringes and allowed to cool. This formulation can be injected through an 18 gauge needle at 56°C. Rapamycin

| Drug | Manufacturer | Concentration in Film |
|---|---|---|
| Sodium Hyaluronate Pharma Grade 150 | FMC BioPolymer | |
| Fucoidan | Sigma | 33% w/w |
| Rapamycin | AG Scientific | 1.6% w/w |

To measure drug release from the PCL paste, 10 mg aliquots of molten paste are injected onto the base of 15 mL glass tubes and allowed to cool and set. Fifteen mL of phosphate buffered saline (PBS) are added to the tubes and the tubes are capped, and tumbled end over end in a 37°C oven. At specified times, the tubes are removed and the amount of drug released is analyzed by absorbance spectroscopy. The release of rapamycin is characterized by an initial burst of drug release followed by a slow sustained release. This dosage form of rapamycin represents a biocompatible, biodegradable, injectable formulation of inhibitor that releases the drug in a controlled manner.

### EXAMPLE 9: THE EFFECT OF RAPAMYCIN-LOADED PELLETS ON ANGIOGENESIS IN THE CHORIOALLANTOIC MEMBRANE OF THE CHICK EMBRYO (CAM ASSAY)

Fertilized chicken eggs are obtained from a local hatchery and placed in an incubator with an automatic rotator at 37° C for 3 and ½ days. The eggs are manually rotated in the incubator such that their sharp end is facing up for 5-10 minutes to allow detachment of the egg contents from the inner membrane. Using 70% ethanol and Kimwipes, the entire eggshell is wiped down to help clean and sanitize the outside of the egg. Inside a laminar flow hood, the egg is held with the blunt side up and a hole is made in the blunt end of the egg by carefully cracking the shell with the end of forceps. The shell remnants are gently removed with forceps to form a hole in the blunt end. This circular hole can be made as large as 2 to 3 cm in diameter without damaging the inner membrane. Once the hole is created in the shell, the inner shell membrane (which houses the egg contents) is gently torn and removed using the forceps, taking care not to damage the chorioallantoic membrane (CAM) (which houses the yolk and developing chick embryo).

The hole is then covered with the sheet of sterilized parafilm wax paper by gently stretching the parafilm and placing it around the hole. The egg is then placed in the egg rack in the incubator (37° C) and positioned in such a way as to prevent rotation. After 6 days each egg is removed one by one from the incubator (blunt side up), and the parafilm covering the window is removed for direct access to the CAM, which originates from the hindgut of the embryo. Polymeric pellets containing rapamycine (loaded between 1% - 30% w/w) are placed onto the growing capillary bed of the CAM. The egg contents are then resealed with a parafilm sheet and the egg is placed back into the 37° C incubator. After 2 more days, analysis of the CAM vasculature is recorded (48 hours after placing the drug onto the CAM capillary bed). The effect of the drug on the CAM is rated using an avascular scale, which grades the effect of the drug as 0, 1, 2, or 3. The values of the avascular scale describe the following:

| | |
|---|---|
| 0 | No antiangiogenic activity |
| 1 | Microvessel reduction |
| 2 | Small avascular zone measuring the size of the drug pellet (2mm in diameter) |
| 3 | Avascular zone measuring 4-5mm in diameter. |

The presence of the rapamycin-loaded pellet prevents or decreases angiogenesis in the CAM assay, which demonstrates an antiangiogenic activity of rapamycin and show that a polymeric slow release formulation of rapamycin is an effective method of releasing therapeutically effective concentrations of the drug without inducing undue toxicity.

### EXAMPLE 10: ENCAPSULATION OF RAPAMYCIN IN CHITOSAN FILMS

Rapamycin is dissolved in 1.2 mL of dimethyl sulphoxide and then pipetted into 4 mL of a 2.5% w/v chitosan (Fluka scientific, low molecular weight) solution in 2% w/v acetic acid. This mixture is stirred by spatula for five minutes to homogeneously suspend the precipitated drug in the chitosan solution. Four mL of this viscous mixture is then poured into 2.5 cm plastic petri dishes and dried at 37° C overnight. The chitosan dries to thin films that are removed from the petri dishes. These films are moderately flexible, about 35 mm thick and with the inhibitor suspended uniformly in the chitosan matrix at a concentration of 10% (w/w relative to chitosan). To measure drug release from these chitosan films, 20 mg pieces are placed into 10 mL of PBS pH 7.4 in capped tubes and tumbled for specific times at 37° C. The amount of rapamycin r released from the films into the PBS is measured by absorbance at 260 nm. The release of the drug is characterized by an initial burst followed by a slow sustained release. This dosage form of rapamycin represents a biocompatible, mucoadhesive formulation of the drug that releases the drug in a controlled manner.

### EXAMPLE 11: EFFICACY OF FUCOIDAN LOADED (33% W/W) HYALURONIC ACID FILM AND FUCOIDAN INSTILLATE (3% W/V) IN RINGERS LACTATE SOLUTION FOR THE PREVENTION OF SURGICAL ADHESIONS USING OF THE UTERINE HORN SURGICAL ADHESION MODEL IN RABBITS.

A 33% fucoidan film was formulated by dissolving hyaluronic acid (HA) with glycerol in distilled water. The dissolution of the HA occurred within 2 hours of being tumbled end-over-end in 37° C oven. Fucoidan (Sigma Chemicals) was added to the HA/glycerol solution with mixing. EDAC, a cross-linking agent, was added to the formulation with vigorous mixing. This solution was then cast in plastic petri dishes, which were then placed in a 60° C oven overnight to dry the formulation down to a film. The resulting film (33% fucoidan w/w) was then removed from the petri dish with tweezers and cut to appropriate size.

A 3% w/v fucoidan instillate was formulated. The measured amount of fucoidan was mixed with Ringer's Lactate solution to form the 3% w/v instillate.

These two formulations were tested using a uterine horn surgical adhesion model in rabbits. Briefly, an incision was made in the abdomen of the rabbits. The uterine horns were located and injured by clamping near the base of the horn for a prescribed (and consistent) length of time. The peritoneal sidewall of the rabbit was injured in a specified area by abrasion with a scalpel. The uterine horns were then placed in such a way that they lay on the abraded area of the peritoneal sidewall and stitched at the tip of the horn. The stitch was outside the abraded area of the sidewall but prevented the uterine horn from contracting away from the abraded sidewall area.

The efficacy of the fucoidan film formulations was evaluated by placing the film directly on the abraded sidewall area (between the horn and the sidewall). Note that the horn was not held tightly to the sidewall and so the film placed on the injured sidewall was mucoadhesive and had to remain in place due to its own physical properties.

The efficacy of the fucoidan instillate formulation was evaluated by instilling 30 mL of the formulation into the abdomen of the rabbit prior to completing the surgical procedure.

These formulations were compared with a control group which was treated by the instillation of 30 mL of Lactated Ringer's Injection USP into the abdomen of the rabbits prior to completing the surgical procedure.

At 14 days after the procedure the rabbits were euthanized and the extent of adhesion formation was evaluated. The evaluator was blinded as to which group was being evaluated. These adhesions were rated as a product of the area covered by the adhesions and the strength of the adhesions that formed. The area covered by adhesions was rated on a 4 point scale and the strength of the adhesions was rated on a scale of 0 to 3. The following scales were used:
Strength of Adhesion Rating Scale:

| | |
|---|---|
| 0 | no adhesions |
| 1 | adhesions separable by blunt dissection |
| 2 | adhesions not easily separable |
| 3 | sharp dissection of adhesion required (tearing of the wall or horn) |

Area Covered by Adhesions Scale:

| | |
|---|---|
| 1-25% | 1 |
| 25-50% | 2 |
| 51-75% | 3 |
| 76-100% | 4 |

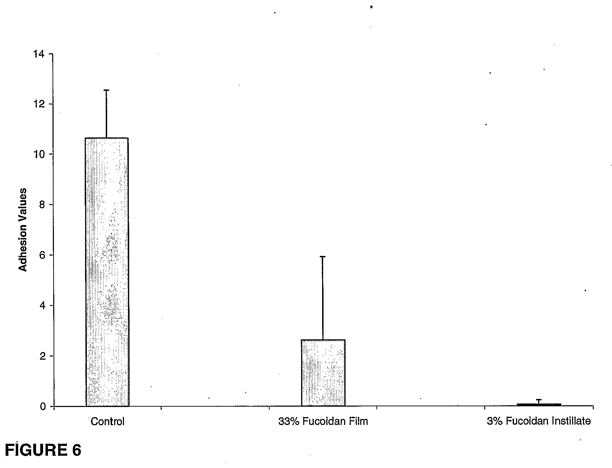

An adhesion score for each rabbit is then obtained by multiplying the scores for the strength of the adhesions by the scores for the area covered by the adhesion. The results of the adhesion scores for the respective test groups are shown in FIG. 6. The data represent the average adhesion score from 8 animals (± 1 S.D.).

These data demonstrate that both of the fucoidan formulations tested reduced the average adhesion values of the rabbits in the group, compared with control, and were thus demonstrated to be effective in the treatment of surgical adhesions, and that the instillate appeared to be more efficacious than the film.

### EXAMPLE 12: EFFICACY OF FUCOIDAN GEL FORMULATIONS FOR THE PREVENTION OF SURGICAL ADHESIONS USING THE CAECUM SIDEWALL SURGICAL ADHESION MODEL IN RATS

A series of gels were formulated for this experiment:
1. 0% w/v Fucoidan in 5.5% w/v Hyaluronic acid gel
2. 1.5% w/v Fucoidan in 5.5% w/v Hyaluronic acid gel
3. 3% w/v Fucoidan in 5.5% w/v Hyaluronic acid gel
4. 6% w/v Fucoidan in 5.5% w/v Hyaluronic acid gel

These gels were evaluated using the caecum sidewall surgical adhesion model in rats to determine their efficacy for the prevention of surgical adhesions.

Briefly, an incision was made along the abdomen of the rat and the caecum was located and pulled out. The caecum was scraped 15 times up, 15 times down and then 15 times up with a scalpel blade. The peritoneal wall was separated from the skin, and a small square of this wall had the top membrane and one fiber layer of muscle tissue removed. The caecum was then stitched in place to cover the square. The stitches were started in the two top corners of the caecum but not completed so the caecum was not "tied down" to the injured peritoneal wall. The prescribed treatment was then applied as a specific gel was placed between the caecum and the peritoneal wall. In the case of the control group, no treatment formulation was applied. The stitches were completed to tie down the corners of the caecum to the wall.

At 7 days after the procedure the rats were euthanized and the extent of adhesion formation was evaluated between the caecum and the sidewall. The evaluator was blinded as to which group was being evaluated. These adhesions were rated as a product of the area covered by the adhesions and the strength of the adhesions that formed. The area covered by adhesions was rated on a 4 point scale and the strength of the adhesions was rated on a scale of 0 to 3. The following scales were used:
Strength of Adhesion Rating Scale:

| | |
|---|---|
| 0 | no adhesions |
| 1 | adhesions separable by blunt dissection |
| 2 | adhesions not easily separable |
| 3 | sharp dissection of adhesion required (tearing of the wall or horn) |

Area Covered by Adhesions Scale:

| | |
|---|---|
| 1-25% | 1 |
| 25-50% | 2 |
| 51-75% | 3 |
| 76-100% | 4 |

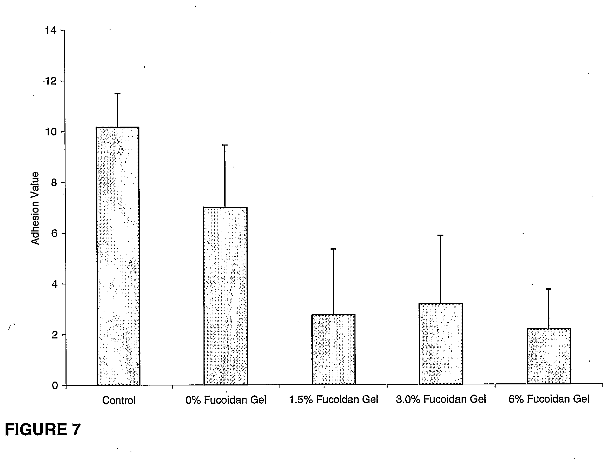

An overall adhesion score for each rat is then obtained by multiplying the scores for the strength of the adhesions by the scores for the area covered by the adhesion. The results of the adhesion scores for the respective test groups are shown in Figure 7. These results indicate that the average overall adhesion scores for the animals in the treatment groups (fucoidan gel groups) were significantly lower than those from the control group. This demonstrates that the fucoidan gel formulations are effective in preventing the formation of fibrous adhesions.

### EXAMPLE 13: EFFICACY OF FUCOIDAN INSTILLATE FORMULATIONS FOR THE PREVENTION OF SURGICAL ADHESIONS USING THE UTERINE HORN SURGICAL ADHESION MODEL IN RATS

Fucoidan instillate solutions of concentrations 0.001%, 0.003% and 0.01% w/v were manufactured by dissolving appropriate amounts of fucoidan in Lactated Ringer's Injection USP to achieve the stated concentrations. The fucoidan was an extract from the brown marine algae *Fucus vesiculosis* and was obtained from Sigma Chemicals. These formulations were evaluated for their efficacy in the prevention of surgical adhesions in rats using the rat uterine horn surgical adhesion model, with Lactated Ringer's Injection USP as a control.

The procedure for the rat uterine horn model was as follows. Each rat was anesthetized and given an antibiotic. A 3-4 cm incision was then made along the midline of the abdomen and the linea alba of the peritoneal wall. One of the uterine horns was located, and the horn was devascularized and excised from the mesentery. It was scraped 15 times up, 15 times down and then 15 times up with a scalpel blade. This produced petechial hemorrhaging and the same process was repeated on the contralateral horn. The peritoneal wall was separated from the skin and inverted, exposing the inside of the wall, and a small region (1.0 x 2.5 cm) of the peritoneum was excised. The uterine horn was then positioned over this sidewall wound and sutured loosely. The same procedure was performed on the contralateral sidewall. The incision was closed using 5-0 sutures for the peritoneal sidewall. Immediately before the last stitch was tied off, 5 mL of the instillate to be tested was deposited in the abdominal cavity using a sterilized pre-loaded syringe. To complete the surgery, 3-0 sutures were used for the skin.

After 7 days the rats were euthanized and their adhesions were examined. The peritoneal wall was inverted and the adhesions between the uterine horn and the sidewall were examined.

Adhesions were scored on basis of adhesion strength and the estimated area covered by the adhesions. A score was generated for each parameter using the following scales:
Strength of Adhesion Rating Scale:

| | |
|---|---|
| 0 | no adhesions |
| 1 | adhesions separable by blunt dissection |
| 2 | adhesions not easily separable |
| 3 | sharp dissection of adhesion required (tearing of the wall or horn) |

Area Covered by Adhesions Scale:

| | |
|---|---|
| 1-25% | 1 |
| 25-50% | 2 |
| 51-75% | 3 |
| 76-100% | 4 |

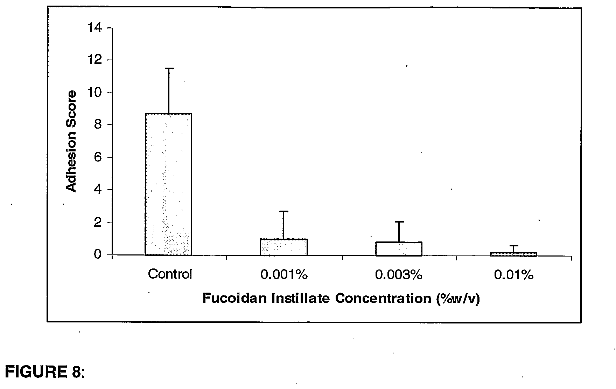

The overall adhesion score for each rat was then obtained by multiplying the score for the strength of the adhesions by the score for the area covered by the adhesion. Five animals were evaluated for each treatment group and four rats were in the control group. A graph of the average overall adhesion scores for each group is given in FIG. 8. These data show that the overall adhesion scores are significantly reduced by the presence of fucoidan in the instillate at all concentrations tested, and demonstrate that fucoidan is effective in preventing the formation of fibrous adhesions.

### EXAMPLE 14: EFFICACY OF FUCOIDAN INSTILLATE FORMULATIONS FOR THE PREVENTION OF SURGICAL ADHESIONS USING THE UTERINE HORN SURGICAL ADHESION MODEL IN RABBITS

Efficacy studies conducted in rabbits utilized the uterine horn model of surgical adhesion disease in rabbits. New Zealand White rabbits were obtained and housed for at least 3 days prior to treatment. The rabbits had access to a diet of rabbit chow and water *ad libitum.* The procedure was as follows:
The rabbits were weighed and then prepared for surgery by premedication with 22.5 mg/kg ketamine and 2.5 mg/kg xylazine given intramuscularly in the flank of the hind leg. A nose cone with 5% isoflurane and oxygen inhalation was used for anesthetic induction. The rabbit was then intubated and the animal maintained on isoflurane for the remainder of the procedure. Duratears were added under each eyelid to prevent the eyes from drying.

The abdomen and a portion of the back of the rabbit were then shaved and the animal transferred to the surgical table in the operating room. The abdomen was cleaned, draped with sterile towels and entered via a midline abdominal incision. One of the uterine horns was located and severed. The proximal 5 cm of the uterine horn was devascularized using an electrocauterizer. The devascularized portion of the horn was excised from the broad uterine ligament and placed on sterile gauze damped with saline. The abdominal wall was retracted and everted to expose a section of the parietal peritoneum nearest the natural resting uterine horn location. The peritoneum and the exposed superficial layer of muscle (transverses abdominis) were excised over an area of 1.5 x 3 cm². Excision included portions of the underlying internal oblique muscle, leaving behind some intact and some torn fibres from the second layer. Minor local bleeding was tamponaded until controlled. In animals treated with film formulations the film was placed directly on the abraded peritoneum (between the horn and the sidewall). The devascularized section of the uterine horn was positioned over the sidewall wound and sutured with a single stitch at a point at least one centimeter distal to the superior and inferior margins of the abraded site. The procedure was repeated on the contralateral uterine horn and sidewall.

Following the surgical procedure, the abdominal wall was closed with 4-0 silk sutures. The skin was then closed with 3-0 silk sutures. Prior to the last stitch being tied, 30 mL of instillate solution was administered into the abdomen of treated rabbits. The last stitch was then tied taking care to ensure that no instillate leaked from the abdominal cavity of the animals. Rabbits were placed on clean bedding under a heat lamp and covered with towels to maintain body temperature during recovery.

At 14 days after the procedure the animals were euthanized and the extent of adhesion formation was evaluated between the injured uterine horn and peritoneum. Any abdominal adhesions that have formed were also noted. The evaluator was blind as to which formulation was being evaluated. Uterine horn adhesions were calculated as a product of the area covered by the adhesions and the strength of the adhesions that formed, using the following scale:
Strength of Adhesion Rating Scale:

| | |
|---|---|
| 0 - 0.5 | unusually free |
| 0.5 - 1.5 | separable by blunt dissection |
| 1.5 - 2 | not easily separable in single area |
| 2 - 3 | sharp dissection required |
| 3 | perforation or tearing is unavoidable |

Area of Adhesions Scale:

| | |
|---|---|
| 1-25% | 1 |
| 25-50% | 2 |
| 51-75% | 3 |
| 76-100% | 4 |

The total adhesion score for a given treatment is reported as the product of the strength score and the area of adhesions score. Using this scale the maximum score for an adhesion is 12.

### Efficacy Experiment

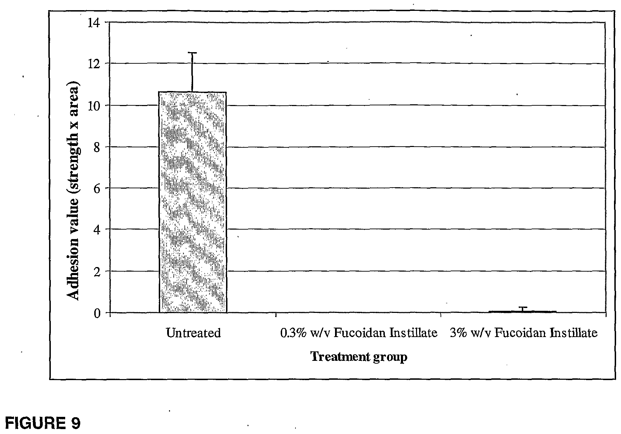

A total of 19 rabbits were used in this experiment. All rabbits were subjected to the uterine horn procedure described above. The rabbits were divided into 3 groups, with 8 animals untreated, 3 receiving 30 mL of 0.3% w/v fucoidan instillate solution (90 mg fucoidan dose), and 8 animals receiving 30 mL of 3% w/v fucoidan instillate solution (900 mg fucoidan dose). The adhesions scores in each animal were evaluated 14 days after surgery and are plotted in Figure 9.

The data show that treatment with fucoidan instillate resulted in a dramatic decrease in the overall adhesion score relative to the untreated control. These data show that fucoidan is effective at inhibiting or preventing surgical adhesions.

### EXAMPLE 15: THE USE OF FUCOIDAN FROM FUCUS VESICULOSIS AND LAMINARIA JAPONICA (KOMBU) FOR PREVENTION OF SURGICAL ADHESIONS USING THE UTERINE HORN SURGICAL ADHESION MODEL IN RATS

Fucoidan from both *Fucus vesiculosis* and *Laminaria japonica* (Kombu) was evaluated for efficacy in the prevention of surgical adhesions using the rat uterine horn surgical adhesion model. Each source of fucoidan was dissolved in Lactated Ringer's Injection USP at a concentration of 0.001 % w/v. This was administered to rats as a 5 mL dose given intra-peritoneally following surgery using the uterine horn surgical adhesion model. The efficacy of these formulations was compared to that of a Lactated Ringer's Injection USP control (5 mL per rat).

First the rat was anesthetized and was given an antibiotic. Then a 3-4 cm incision was made along the midline of the abdomen and the linea alba of the peritoneal wall. One of the uterine horns was located, and the horn was devascularized and excised from the mesentery. It was scraped 15 times up, 15 times down and then 15 times up with a scalpel blade. This produced petechial hemorrhage and the same process was repeated on the contralateral horn. The peritoneal wall was separated from the skin and inverted, exposing the inside of the wall, and a small region (1.0 x 2.5 cm²) of the peritoneum was excised. The uterine horn was then positioned over this sidewall wound and sutured loosely, with one stitch distal to and one stitch caudal to the injured peritoneal sidewall wound. The same procedure was performed on the contralateral sidewall.

The surgical incision was closed using 5-0 sutures for the peritoneal sidewall. Immediately before the last stitch was tied off, the instillate to be tested was deposited in the abdominal cavity. The last stitch was then tied off. Closure of the skin incision was performed using 3-0 sutures.

After 7 days the rats were euthanised and their adhesions were examined. The peritoneal wall was inverted the adhesions between the uterine horn and the sidewall were examined.

Adhesions were scored on basis of strength and the area of the abraded sidewall in which adhesions were present. The strength of the adhesions within the abraded area and the area covered by adhesions were scored using the following scales:
Strength of Adhesion Rating Scale:

| | |
|---|---|
| 0 | no adhesions |
| 1 | adhesions separable by blunt dissection |
| 2 | adhesions not easily separable |
| 3 | sharp dissection of adhesion required (tearing of the wall or horn) |

Area Covered by Adhesions Scale:

| | |
|---|---|
| 1-25% | 1 |
| 25-50% | 2 |
| 51-75% | 3 |
| 76-100% | 4 |

An adhesion score for each rat was then obtained by multiplying the scores for the strength of the adhesions by the scores for the area covered by the adhesion. Rats that had undergone this procedure were divided into three treatment groups (n=5 per group) and received 5 mL of either 0.001% w/v instillate fucoidan from *Fucus vesiculosis,* 0.001% w/v instillate fucoidan from *Laminaria japonica*

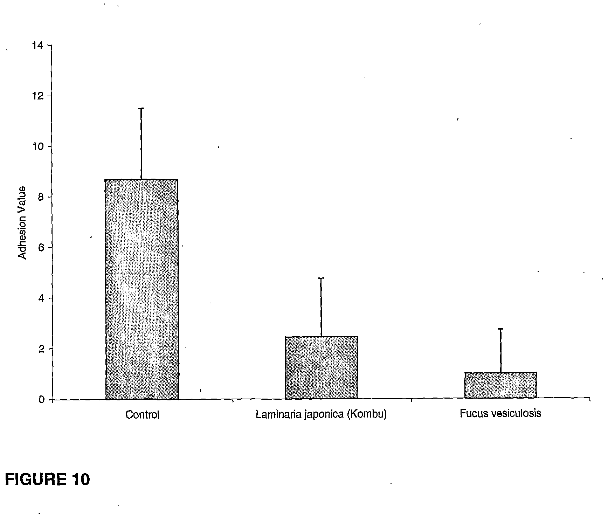

(Kombu), or Lactated Ringer's Injection USP (control). The effect on the average adhesion scores for these three groups is given in FIG. 10. These data demonstrate, by virtue of significantly lower adhesion scores from the treated groups relative to control, that fucoidan from either source is effective in the prevention of surgical adhesions.

### EXAMPLE 16: THE USE OF FUCOIDAN FROM FUCUS VESICULOSUS (LOW SULPHATE CONTENT) AND LAMINARIA JAPONICA (HIGH SULPHATE CONTENT) FOR PREVENTION OF SURGICAL ADHESIONS USING THE CAECAL-SIDEWALL SURGICAL ADHESION MODEL IN RATS

Fucoidan from both *Fucus vesiculosis* and *Laminaria japonica* (Kombu) was evaluated for efficacy in the prevention of surgical adhesions using the rat caecal-sidewall surgical adhesion model. Physical characterization of fucans was carried out using a variety of techniques. The total carbohydrate content of the fucan samples was done using the Phenol-Sulphuric method using fucose as the standard (Smith et. al. Anal Chem. 28: 350 (1956)). This method used 1 mL of sample mixed with 1 mL of freshly prepared 5% phenol in a test tube, to which 5 mL of concentrated sulphuric acid was added as rapidly as possible so that the mixture boiled. Thorough mixing was ensured and the optical density of the solution was measured after 30 - 45 minutes in a spectrophotometer at 480 nm. This method was calibrated using solutions of known fucose concentration to create a standard curve. Determination of fucose content was achieved through hydrolysis of the sample using trifluoracetic acid (TFA), following the method specified for oligosaccharides (50 µL of sample solution plus 50 µL of 4N TFA for 5 hours at 98° C). Determination of the fucose was done by ion chromatography (fucose as standard). Sulphate content in the samples was determined by hydrolysis using 2 M hydrochloric acid at 99° C for 3 hours. The sulphate concentration was measured by isocratic ion chromatography with suppressed conductivity (Stevenson, T.T and Furneaux, R.H. Carbohydrate Research, 210: 277-298 (1991)).

Using a molecular weight for sulphate (SO₄) as 97.1 g/mole and a molecular weight of fucose (C₆H₁₁O₅) of 163.1 g/mole and by measuring the sulphate content, and fucose content of the fucan samples, the ratio of sulphate:fucose could be used to calculate the average number of sulphate groups per fucose monomer. This was done for the two sources of fucoidan and the following values were obtained:

| Fucan source | SO₄ content (% w/w) | Fucose content (% w/w) | SO₄:Fucose weight ratio | Average SO₄ per fucose |
|---|---|---|---|---|
| *F vesiculosus -* 1 | 22.1 | 35 | 0.63 | 1.06 |
| *L. japonica -* 1 | 29.5 | 28 | 1.05 | 1.8 |

Fucoidan films loaded at 33% w/w were formulated by dissolving hyaluronic acid (HA) with glycerol in distilled water. The dissolution of the HA occurred within 2 hours of being tumbled end-over-end in 37° C oven. Fucoidan (Sigma Chemicals or Takara Bio) was added to the HA/glycerol solution with mixing. EDAC, a cross-linking agent, was added to the formulation with vigorous mixing. This solution was then cast in plastic petri dishes, which were then placed in a 60° C oven overnight to dry the formulation down to a film. The resulting film (33% fucoidan w/w) was then removed from the petri dish with tweezers and cut to appropriate size.

These formulations were evaluated in rats as follows:
Animals were randomly assigned to a treatment group, weighed and anesthetized with isofluorane gas. The abdomen was shaved and cleaned with a skin-antibacterial cleanser (Steri-Stat 2%) and wiped with a chlorohexane soaked gauze. A nick was made in one of the tail veins to elicit a small amount of blood (∼100 µL). An antibiotic (40.000 IU/kg of Duplocillin) was injected into the right thigh and an analgesic (0.01 mg/kg of buprenorphine) was injected into the left thigh of each rat.

A 4 cm incision was made in the skin beginning approximately 2 cm caudal to the linea alba while the muscle was tended with forceps. The caecum was located and exteriorized. Using a number 10 scalpel held at a 45 degree angle relative to the caecum surface, the caecum was scraped 45 times to abrade the surface. The scraped caecum was wrapped in saline-soaked gauze. Doynes were used to separate the peritoneal wall from the skin, and the peritoneal wall was inverted using the doynes to expose the inside of the wall. A rectangular injury roughly 1.2 cm by 1.8 cm was made by shallow incisions to the peritoneal wall. The top membrane and a layer of muscle tissue was removed using forceps. The caecum was stitched to the four corners of the rectangle using a 5-0 suture, and without tying off the top two stitches. A piece of film was placed onto the abraded rectangle and then the top two stitches were tied firmly. In the case of the control group, no film was placed over the abraded site.

The exposed organs were then replaced in the abdomen in such a way as to prevent torsional stress on the intestines. The abdominal wall was closed with 5-0 sutures and the surgical incision was closed with 3-0 sutures. The external incision was wiped with a chlorohexane soaked gauze. A collar was placed around the neck of the animal to prevent it from interfering with the stitches. The rat was placed in a clean cage and warmed with a heating lamp until consciousness was regained.

The adhesions that developed in the rats were evaluated 7 days after surgery. Each animal was euthanized with CO₂ and the external incision was visually inspected for signs of inflammation or lack of healing. The animal was reopened in an arch around the midline and the internal organs were visually checked for anomalies, and any abdominal adhesions were noted. The peritoneal wall was inverted and adhesions present between the abraded caecum and sidewall were evaluated. The sutures were cut and fibrous adhesions between the caecum and the sidewall as well at the stitching points are assessed arid scored according to a predefined scoring system. There are two criterion used in this assessment. First, percentage of the total abraded area covered by fibrous adhesions was determined according to the following scale:

| Area Score | % of abraded area covered by fibrous adhesion |
|---|---|
| 1 | 0 - 25% |
| 2 | 26 - 50% |
| 3 | 51 - 75% |
| 4 | 76 - 100% |

The strength of the observed fibrous adhesions was rated according to the following numerical scale:

| Strength Score | Strength of fibrous adhesions |
|---|---|
| 0 - 0.5 | Unusually free of fibrous adhesions |
| 0.5 - 1.5 | Fibrous adhesions separable by blunt dissection |
| 1.5 - 2 | Fibrous adhesions not easily separable |
| 2 - 3 | Shard dissection required, tearing unavoidable |

The overall fibrous adhesion score for an animal was determined by multiplying the area score by the strength of fibrous adhesion score (Max adhesion score is 12).

The results obtained were as follows:

| Fucoidan source | Average SO₄ per fucose | Mean Adhesion score (± 1 S.D.) at 33% w/w loading |
|---|---|---|
| F. vesiculosus -1 | 1.06 | 0.5 ± 0.5 (n=3) |
| L. japonica - 1 | 1.8 | All died within 24 hours of treatment due to internal hemorrhaging |

The mean adhesion score of 0.5 from the formulation loaded with the fucoidan from *F. vesiculosus* indicates that this fucan was effective in this model at preventing the formation of surgical adhesions. Rats that had undergone this procedure and were treated with film alone (no fucoidan) typically developed adhesions with scores of between 6 and 10 (data not shown).

### EXAMPLE 17: THE USE OF FUCOIDAN FROM FUCUS VESICULOSUS (LOW SULPHATE CONTENT) AND UNDARIA PINNATIFIDA (HIGH SULPHATE CONTENT) FOR THE PREVENTION OF SURGICAL ADHESIONS USING THE UTERINE HORN SURGICAL ADHESION MODEL IN RABBITS

Fucoidan from both *Fucus vesiculosis* and *Laminaria japonica* (Kombu) was evaluated for efficacy in the prevention of surgical adhesions using the rat caecal-sidewall surgical adhesion model. Physical characterization of fucans was carried out using a variety of techniques. The total carbohydrate content of the fucan samples was done using the Phenol-Sulphuric method using fucose as the standard (Smith et. al. Anal Chem. 28: 350 (1956)). This method used 1 mL of sample mixed with 1 mL of freshly prepared 5% phenol in a test tube, to which 5 mL of concentrated sulphuric acid is added as rapidly as possible so that the mixture boils. Thorough mixing was ensured and the optical density of the solution was measured after 30 - 45 minutes in a spectrophotometer at 480 nm. This method was calibrated using solutions of known fucose concentration to create a standard curve. Determination of fucose content was achieved through hydrolysis of the sample using trifluoracetic acid (TFA), following the method specified for oligosaccharides (50 µL of sample solution plus 50 µL of 4N TFA for 5 hours at 98° C). Determination of the fucose was done by ion chromatography (fucose as standard). Sulphate content in the samples was determined by hydrolysis using 2 M hydrochloric acid at 99° C for 3 hours. The sulphate concentration was measured by isocratic ion chromatography with suppressed conductivity.

Using a molecular weight for sulphate (SO₄) as 97.1 g/mole and a molecular weight of fucose (C₆H₁₁O₅) of 163.1 g/mole and by measuring the sulphate content, and fucose content of the fucan samples, the ratio of sulphate:fucose could be used to calculate the average number of sulphate groups per fucose monomer. This was done for three sources of fucoidan and the following values were obtained:

| Fucan source | SO₄ content (% w/w) | Fucose content (% w/w) | SO₄: Fucose weight ratio | Average SO₄ per fucose |
|---|---|---|---|---|
| *F vesiculosus -* 1 | 22.1 | 35 | 0.63 | 1.06 |
| *F vesiculosus -* 2 | 27.7 | 43 | 0.64 | 1.07 |
| *U pinnatifida* | 25.8 | 24 | 1.07 | 1.8 |

These fucoidan samples were each dissolved in Lactated Ringer's Injection USP at a concentration of 1% w/v to make a fucoidan loaded instillate formulation. Each of these formulations was administered to rabbits that had undergone the uterine horn surgical adhesion model described as follows:

The rabbits were weighed and then prepared for surgery by premedication with 22.5 mg/kg ketamine and 2.5 mg/kg xylazine given intramuscularly (IM) in the flank of the hind leg. A nose cone with 5% isoflurane and oxygen inhalation was used for anesthetic induction. The rabbit was then intubated and the animal maintained on isoflurane for the remainder of the procedure. Duratears were added under each eyelid to prevent the eyes from drying.

The abdomen and a portion of the back of the rabbit were then shaved and the animal transferred to the surgical table in the operating room.

All instruments were sterile and a sterile field was maintained throughout the surgeries. Rabbits were placed on the operating table and the anesthetic machine was attached. Anesthesia was induced and maintained using an appropriate combination of isofluorane and oxygen. A conducting gel was placed on the shaved portion of the rabbit's back, and the rabbit was placed on the electrocauterizing plate on the surgical table. All four legs of the rabbit were secured with ties to the operating table. An antibiotic, Duplocillin (40,000 IU/kg) was administered IM in one hind leg. Buprenorphine (0.01 mg/kg) was administered IM in the contralateral leg, and was sufficient to provide effective analgesia throughout the post-surgical recovery period. Approximately 0.5 ml of blood was taken from the artery of the ear using a 22G IV catheter and syringe. The animal was tattooed in the contralateral ear with a number, which was recorded.

The abdomen was cleaned three times using Dexidine, sterilized with Chlorhexadine, draped with sterile towels and entered via a midline abdominal incision. One of the uterine horns was located, and the uterine tube was severed at its caudal extremity. The proximal 5 cm of the uterine horn was devascularized using the electrocauterizer. Next the horn was excised from the broad uterine ligament, and using a number 10 scalpel held at a 45 degree angle relative to the horn surface, each horn was scraped separately 45 times to abrade the surface. Each scraped horn was wrapped in saline-soaked gauze. The abdominal wall was then retracted and everted to expose a section of the parietal peritoneum nearest the natural resting uterine horn location. The peritoneum and the exposed superficial layer of muscle (transverses abdominis) were excised over an area of 1.0 x 3 cm². Excision included portions of the underlying internal oblique muscle, leaving behind some intact and some torn fibres from the second layer. Minor local bleeding was tamponaded until controlled. The devascularized section of the uterine horn was positioned over the sidewall wound and sutured with a single stitch at a point at least one centimeter distal to the superior and inferior margins of the abraded site. The procedure was repeated on the contralateral uterine horn and sidewall.

Following the surgical procedure, the abdominal wall was closed with 5-0 silk sutures, leaving a small opening for the introduction of an 18-gauge needle. If instillate was used a 30 mL volume of the anti-adhesion solution was slowly instilled by syringe into the abdomen. The opening was then closed, and the incision examined for any leakage. The skin was then closed with 3-0 silk sutures. Subjects were placed on clean bedding, under a heat lamp, and covered with towels to maintain body temperature during recovery. When completely mobile, the rabbits were returned to their room, provided with food and water *ad libitum,* and examined daily for signs of wound infection and any indications of morbidity.

After 14 days the adhesions that had formed in each animal was evaluated as follows:
Each animal was euthanized with 1.5 mL of 120 mg/ml of pentobarbital sodium (Euthanyl®) injected into the ear vein and the external incision was visually inspected for signs of inflammation or lack of healing. The animal was reopened in an arch around the midline and the internal organs were visually checked for anomalies, and any abdominal adhesions were noted. Each abdominal adhesion was assigned a strength score according to the table below. The peritoneal wall was inverted and adhesions present between the abraded uterine horn and sidewall were evaluated. The sutures were cut and fibrous adhesions between the uterine horn and the sidewall were assessed and scored according to a predefined scoring system. There were two criterion used in this assessment. First, percentage of the total abraded area covered by fibrous adhesions was determined according to the following scale:

| Area Score | % of abraded area covered by fibrous adhesion |
|---|---|
| 1 | 0 - 25% |
| 2 | 26 - 50% |
| 3 | 51 - 75% |
| 4 | 76 - 100% |

The strength of the observed fibrous adhesions was rated according to the following numerical scale:

| Strength Score | Strength of fibrous adhesions |
|---|---|
| 0 - 0.5 | Unusually free of fibrous adhesions |
| 0.5 - 1.5 | Fibrous adhesions separable by blunt dissection |
| 1.5 - 2 | Fibrous adhesions not easily separable |
| 2 - 3 | Shard dissection required, tearing unavoidable |

The overall fibrous adhesion score for an animal was determined by multiplying the area score by the strength of fibrous adhesion score.

The results obtained were as follows:

| Fucoidan source / formulation | Average SO4 per fucose | Mean Adhesion score (± 1 S.D.) |
|---|---|---|
| *F. vesiculosus* - 1 | 1.06 | 2.67 ± 3.06 (n = 3) |
| *F. vesiculosus* - 2 | 1.07 | 4.0 ± 4.62 (n = 4) |
| *U. pinnatifida* | 1.8 | All died within 24 hours of treatment due to internal hemorrhaging |
| Control (0% fucoidan) | NA | 12 ± 0 (n = 4) |

Both fucoidan sources from *F vesiculosus* were shown to be effective (adhesion scores obtained were less than for the control group).

## Claims

1. An agent for use in inhibiting a fibrous adhesion in an animal comprising a sulphate fucan having a sulphate to fucose ratio of less than 1.0.

2. An agent for use according to claim 1, wherein the agent is for use in inhibiting an inflammatory disease in an animal.

3. The agent for use according to claim 1 or 2, wherein the sulphate fucan is in the form of an instillate.

4. The agent for use according to any one of claims 1 to 3, wherein the animal is a human being.

5. The agent for use according to any one of claims 1 to 4, wherein the sulphate fucan has a sulphate to fucose ratio of less than 0.9.

6. The agent for use according to any one of claims 1 to 5, wherein the sulphate fucan is sulphate fucoidan.

## Patentansprüche

1. Mittel zur Verwendung bei der Hemmung einer fibrösen Adhäsion bei einem Tier, umfassend ein Sulfat-Fucan mit einem Sulfat-zu-Fucose-Verhältnis von weniger als 1,0.

2. Mittel zur Verwendung nach Anspruch 1, wobei das Mittel zur Verwendung bei der Hemmung einer Entzündungskrankheit bei einem Tier bestimmt ist.

3. Mittel zur Verwendung nach Anspruch 1 oder 2, wobei das Sulfat-Fucan in Form eines Instillats vorliegt.

4. Mittel zur Verwendung nach einem der Ansprüche 1 bis 3, wobei das Tier ein Mensch ist.

5. Mittel zur Verwendung nach einem der Ansprüche 1 bis 4, wobei das Sulfat-Fucan ein Sulfat-zu-Fucose-Verhältnis von weniger als 0,9 aufweist.

6. Mittel zur Verwendung nach einem der Ansprüche 1 bis 5, wobei das Sulfat-Fucan Sulfat-Fucoidan ist.

## Revendications

1. Agent destiné à être utilisé pour inhiber une adhésion fibreuse chez un animal, comprenant un sulfate de fucane ayant un rapport sulfate sur fucose inférieur à 1,0.

2. Agent destiné à être utiliser selon la revendication 1, dans lequel l'agent est destiné à être utilisé pour inhiber une maladie inflammatoire chez un animal.

3. Agent destiné à être utiliser selon la revendication 1 ou 2, dans lequel le sulfate de fucane est sous la forme d'un instillat.

4. Agent destiné à être utiliser selon l'une quelconque des revendications 1 à 3, dans lequel l'animal est un être humain.

5. Agent destiné à être utiliser selon l'une quelconque des revendications 1 à 4, dans lequel le sulfate de fucane a un rapport sulfate sur fucose inférieur à 0,9.

6. Agent destiné à être utiliser selon l'une quelconque des revendications 1 à 5, dans lequel le sulfate de fucane est le sulfate de fucoïdane.
